# EUROPEAN PATENT SPECIFICATION

(11) **EP 1 941 031 B1**
(45) Date of publication and mention of the grant of the patent: **25.12.2013**
(21) Application number: 06825367.3
(22) Date of filing: 03.10.2006
(51) Int. Cl.: C12N 5/073, C12N 5/071

(54) **ISOLATED EMBRYONIC-LIKE STEM CELLS DERIVED FROM HUMAN UMBILICAL CORD BLOOD**
ISOLIERTE EMBRYONENSTAMMZELLEN AUS MENSCHLICHEM NABELSCHNURBLUT
CELLULES SOUCHES DE TYPE EMBRYONNAIRE ISOLÉES DÉRIVÉES DU SANG DU CORDON OMBILICAL HUMAIN

(30) Priority: 05.10.2005 US 724328 P
(43) Date of publication of application: 09.07.2008
(73) Proprietor: THE BOARD OF TRUSTEES OF THE UNIVERSITY OF ILLINOIS, Urbana, IL 61801 (US); Zhao, Yong, Lisle, IL 60532 (US); Mazzone, Theodore, Wilmette, IL 60091 (US)
(72) Inventor: ZHAO, Yong, IL 60532 (US); MAZZONE, Theodore, IL 60091 (US)
(74) Representative: Lock, Graham James
(86) International application number: PCT/US2006/038524
(87) International publication number: WO 2007/044314

(56) References cited:
- BUZANSKA L ET AL: "Human cord blood-derived cells attain neuronal and glial features in vitro" JOURNAL OF CELL SCIENCE, CAMBRIDGE UNIVERSITY PRESS, LONDON, GB, vol. 115, no. 10, 15 May 2002 (2002-05-15), pages 2131-2138, XP002298410 ISSN: 0021-9533
- ISHIKAWA F ET AL: "Transplanted human cord blood cells give rise to hepatocytes in engrafted mice" ANNALS OF THE NEW YORK ACADEMY OF SCIENCES, NEW YORK ACADEMY OF SCIENCES, NEW YORK, NY, US, vol. 996, 1 May 2003 (2003-05-01), pages 174-185, XP002981769 ISSN: 0077-8923
- BJÖRKLUND A ET AL: "Cell replacement therapies for central nervous system disorders." NATURE NEUROSCIENCE JUN 2000, vol. 3, no. 6, June 2000 (2000-06), pages 537-544, XP002524953 ISSN: 1097-6256
- GLUCKMAN E ET AL: "HEMATOPOIETIC RECONSTITUTION IN A PATIENT WITH FANCONI'S ANEMIA BY MEANS OF UMBILICAL-CORD BLOOD FROM AN HLA-IDENTICAL SIBLING" NEW ENGLAND JOURNAL OF MEDICINE, THE, MASSACHUSETTS MEDICAL SOCIETY, WALTHAM, MA, US, vol. 321, no. 17, 26 October 1989 (1989-10-26), pages 1174-1178, XP009031078 ISSN: 0028-4793
- LEE OSCAR K ET AL: "Isolation of multipotent mesenchymal stem cells from umbilical cord blood." BLOOD 1 MAR 2004, vol. 103, no. 5, 1 March 2004 (2004-03-01), pages 1669-1675, XP002524954 ISSN: 0006-4971
- DJOUAD F ET AL: "IMMUNOSUPPRESSIVE EFFECT OF MESENCHYMAL STEM CELLS FAVORS TUMOR GROWTH IN ALLOGENEIC ANIMALS" BLOOD, AMERICAN SOCIETY OF HEMATOLOGY, US, vol. 102, no. 10, 15 November 2003 (2003-11-15), pages 3837-3844, XP002468564 ISSN: 0006-4971
- CHANG CHUN-JUNG ET AL: "PLACENTA-DERIVED MULTIPOTENT CELLS EXHIBIT IMMUNOSUPPRESSIVE PROPERTIES THAT ARE ENHANCED IN THE PRESENCE OF INTERFERON-GAMMA" STEM CELLS, ALPHAMED PRESS, DAYTON, OH, US, vol. 24, no. 11, 1 November 2006 (2006-11-01), pages 2466-2477, XP009082428 ISSN: 1066-5099
- LEE M W ET AL: "Mesenchymal stem cells from cryopreserved human umbilical cord blood" BIOCHEMICAL AND BIOPHYSICAL RESEARCH COMMUNICATIONS, ACADEMIC PRESS INC. ORLANDO, FL, US, vol. 320, no. 1, 16 July 2004 (2004-07-16), pages 273-278, XP004519095 ISSN: 0006-291X
- ZHAO Y. ET AL.: 'Identification of stem cells from human umbilical cord bloos with embryonic and hematopoietic characteristics' EXP. CELL RES. vol. 312, 2006, pages 2454 - 2464, XP003018775
- KUCIA ET AL.: 'Morphological and molecular characterization of novel population of CXCR4+ SSEA-4+, Oct-4+ very small embryonic-like cell purified from human cord blood-preliminary report' LEUKEMIA vol. 21, 2007, pages 297 - 303, XP003018776
- YOSIDA ET AL.: 'Human cord blood-derived cells generate insulin-producing cells in vivo' STEM CELLS vol. 23, no. 9, 2005, pages 1409 - 1416, XP003018777
- ADAMSON: 'Cord blood stem cell banking and transplantation' HEMATOPOIETIC STEM CELLS. STEM CELLS vol. 15, no. SUPPL. 1, 1997, pages 57 - 61, XP002982306
- HARRIS D.T.: 'Experience in autologous and allogeneic cord blood banking' J. HEMATOTHERAPY vol. 5, 1996, pages 123 - 128, XP008096935
- SIRCHIA G. ET AL.: 'Placental/umbilical cord blood transplantation' HEMATOLOGICA vol. 84, 1999, pages 738 - 747, XP002226904

## Description

### BACKGROUND OF THE INVENTION:

### Field of the Invention

The present invention is related generally to embryonic-like stem cells isolated from human umbilical cord blood, designated herein as cord blood-stem cells (CB-SC), which display the characteristics of embryonic stem cells and hematopoietic cells. These cells have the capability of proliferation and are able to differentiate to multiple types of cells. In addition, CB-SC display low immunogenicity and immune regulation. In the present invention, these cells are for use in stem cell-based therapies for the treatment of diseases such as Parkinson's disease, diabetes, spinal cord damage, multiple sclerosis (MS) cardiovascular disease, stroke and birth defects.

### Background of the Invention

The increasing prevalence of chronic human diseases, e.g. cardiovascular, disease, diabetes, and neuronal degenerative diseases, presents a challenge to find more effective therapies. Stem cell-based therapy, including embryonic and adult stem cells, provides a rational treatment tool for regenerative medicine and has potential to revolutionize modem therapeutics [A. Vats, R. C. Bielby, N. S. Tolley, R. Nerem, J.M. Polak, Stem cells, Lancet 366 (2005) 592-602; M. A. Hussain, N. D. Theise, Stem-cell therapy for diabetes mellitus, Lancet 364 (2004) 203-205; C. M. Rice, N.J. Scolding, Adult stem cells-reprogramming neurological repair? Lancet 364 (2004) 193-199; L. M. Hoffman, M.K. Carpenter, Characterization and culture of human embryonic stem cells, Nat Biotechnol. 23 (2005) 699-708]. Because of their high potential for self renewal and pluripotent differentiation capability, embryonic stem (ES) cells have become a very active area of investigation [A. Vats, R. C. Bielby, N. S. Tolley, R. Nerem, J.M. Polak, Stem cells, Lancet 366 (2005) 592-602; L. M. Hoffman, M.K. Carpenter, Characterization and culture of human embryonic stem cells, Nat Biotechnol. 23 (2005) 699-708; K. H. Wilan, C.T. Scott, S. Herrera, Chasing a cellular fountain of youth, Nat Biotechnol. 23 (2005) 807-815]. Ethical concerns, however, have limited their availability and practical usefulness [C. Dennis, Check E, 'Ethical' routes to stem cells highlight political divide, Nature 437 (2005) 1076-1077; M. Evans, Ethical sourcing of human embryonic stem cells--rational solutions? Nat Rev Mol Cell Biol. 6 (2005) 663-667]. Leaving aside these ethical concerns, using *in vitro* fertilization (IVF) and altered nuclear transfer (ANT) to generate ES cells is made problematic by the complexity of required technologies [M. Evans, Ethical sourcing of human embryonic stem cells--rational solutions? Nat Rev Mol Cell Biol. 6 (2005) 663-667; D. A. Melton, G. Q. Daley, C. G. Jennings, Altered nuclear transfer in stem-cell research - a flawed proposal, N Engl J Med. 351 (2004) 2791-2792].

Recently, human umbilical cord blood has been used as a source of stem cells to repopulate the hematopoietic system and other organs [J. Bonde, D. A. Hess, J. A. Nolta, Recent advances in hematopoietic stem cell biology, Curr Opin Hematol. 11 (2004) 392-398; K. K. Ballen, New trends in umbilical cord blood transplantation, Blood 105 (2005) 3786-3792; D. A. Peterson, Umbilical cord blood cells and brain stroke injury: bringing in fresh blood to address an old problem, J Clin Invest. 114 (2004) 312-314; V. Silani, L. Cova, M. Corbo, A. Ciammola, E. Polli, Stem-cell therapy for amyotrophic lateral sclerosis, Lancet 364 (2004) 200-202]. Cord blood provides an abundant source for generation of stem cells, including mesenchymal stem cells [K. Bieback, S. Kern, H. Kluter, H. Eichler, Critical parameters for the isolation of mesenchymal stem cells from umbilical cord blood, Stem Cells 22 (2004) 625-634; E. J. Gang, S.H. Hong, J. A. Jeong, S. H. Hwang, S. W. Kim, I. H. Yang, C. Ahn, H. Han, H. Kim, In vitro mesengenic potential of human umbilical cord blood-derived mesenchymal stem cells, Biochem Biophys Res Commun. 321 (2004) 102-108; G. Kogler, S: Sensken, J. A. Airey, T. Trapp, M. Muschen, N. Feldhahn, S. Liedtke, R. V. Sorg, J. Fischer, C. Rosenbaum, S. Greschat, A. Knipper, J. Bender, O. Degistirici, J. Gao, A. I: Caplan, E. J. Colletti, G. Almeida-Porada, H. W. Muller, E. Zanjani, P. Wernet, A new human somatic stem cell from placental cord blood with intrinsic pluripotent differentiation potential, J Exp Med. 200 (2004) 123-135] and monocyte-derived stem cells [Y. Zhao, T. Mazzone, Human umbilical cord blood-derived f-macrophages retain pluripotentiality after thrombopoietin expansion, Exp Cell Res. 310 (2005) 311-318]. Stem cells expressing ES molecular markers have been reported from cord blood after removal of hematopoietic cells (including deletion of all leukocyte common antigen CD45 positive cells) [C. P. McGuckin, N. Forraz, M. O. Baradez, S. Navran, J. Zhao, R. Urban, R. Tilton, L. Denner, Production of stem cells with embryonic characteristics from human umbilical cord blood, Cell Prolif. 38 (2005) 245-55]. However, the scarcity of this previously-described cell population [C. P. McGuckin, N. Forraz, M. O. Baradez, S. Navran, J. Zhao, R. Urban, R. Tilton, L. Denner, Production of stem cells with embryonic characteristics from human umbilical cord blood, Cell Prolif. 38 (2005) 245-55] in cord blood significantly restricts its practical application.

Several other embryonic-like stem cells derived from adult sources rather than embryonic sources have also been disclosed. For example, United States Patent Number 7,045,148, United States Patent Applications Serial Numbers 2005/0148034, 2005/0118715, 2004/0028660, 2003/0235909, 2002/0160510, 2003/0180269 and International Patent Application Number WO 03/068937 disclose embryonic-like stem cells extracted from the placenta or from the umbilical cord blood. United States Patent Application Serial Number 2006/0078993 discloses embryonic-like stem cells derived from the amniotic membrane of umbilical cord. The stem cells disclosed in these patents or patent applications are of mesenchymal origin which do not express the CD45 marker (CD45⁻). In another example, United States Patent Application Serial Number 2006/0147426 discloses stem cells derived from human bone marrow.

The present invention relates to a type of stem cells isolated from human umbilical cord blood, designated herein as cord blood-stem cells (CB-SC). These stem cells are of hematopoeitic (and not mesenchymal) origin as indicated by the high expression of the CD45 marker (CD45⁺). These cells can be isolated and expanded using simple technology. CB-SC share properties with human ES cells and hematopoietic cells, including low immunogenicity, ability to proliferate and the ability to differentiate to multiple types of cells.

These and other aspects and attributes of the present invention will be discussed with reference to the following drawings and accompanying specification.

### BRIEF SUMMARY OF THE INVENTION

Provided by the present invention are isolated embryonic-like stem cells, compositions and methods as defined in the appended claims.

The present invention discloses isolated embryonic-like stem cells from human umbilical-cord blood, designated herein as CB-SC, for use in stem cell-based therapy, which are characterized by: (a) displaying embryonic stem cell characteristics comprising a capacity for proliferation and ability to differentiate to multiple types of cells; (b) displaying hematopoietic cell characteristics comprising CD45 expression; (c) phenotypically distinct from lymphocytes, macrophages and monocytes by being negative for the CD3, CD20, CD11b/Mac-1 and CD14 markers; (d) phenotypically distinct from hematopoietic stem cells by being negative for the CD34 marker; (e) displaying low immunogenicity by expressing major histocompatibility complex (MHC) molecules associated with low immunogenicity, and (f) displaying immune regulation by inhibition of mitogen-stimulated lymphocyte proliferation, for use in stem cell-based therapy. These embryonic-like stem cells are capable of proliferation and are able to differentiate to multiple types of cells.

In a preferred embodiment, the embryonic stem characteristics include having phenotypes of positive for stem cell markers Oct-4 and Nanog. In a further embodiment, the low immunogenicity of these stem cells is characterized by the stem cells not able to stimulate lymphocyte proliferation in an allogeneic mixed lymphocyte reaction. In a further embodiment, the stem cells display immune regulation by inhibition of mitogen-stimulated lymphocyte proliferation and regulation of T cell subsets (CD4⁺ T cells, CD8⁺ T cells, and CD4⁺CD25⁺ regulatory T cells).

The embryonic-like stem cells are capable of differentiating to a variety of cells, which include endothelial-like cells, neuronal-like cells, insulin-producing cells, oligodendrocytes, and megakaryocytes.

The present invention further discloses a composition for use in stem cell-based therapy comprising the isolated embryonic-like stem cells described. The embryonic-like stem cells can be used for treating hyperglycemia in a diabetic mammalian subject by administering the cells to the subject.

The present invention also discloses a method for isolating the embryonic-like stem cells. The method comprises: (a) providing a sample of human umbilical cord blood; (b) removing red cells from the sample to obtain mononuclear cells; (c) culturing the mononuclear cells in a culture medium in a non-tissue culture treated culture vessel; and (d) obtaining a cell population which is attached to the culture vessel. The attached cell population can be detached from the culture vessel by, for example, incubation in lidocaine hydrochloride solution wherein the lidocaine hydrochloride is from about 0.1% to about 5%. Optionally, the attached cells can be detached by further incubating the cells with EDTA solution or EDTA solution containing trypsin (trypsin/EDTA) wherein the EDTA is from about 0.5 mM to about 2.5 mM, and the trypsin is from about 0.05% to about 0.25%. Furthermore, the cell culture does not require a cell feeder.

### BRIEF DESCRIPTION OF THE DRAWINGS:

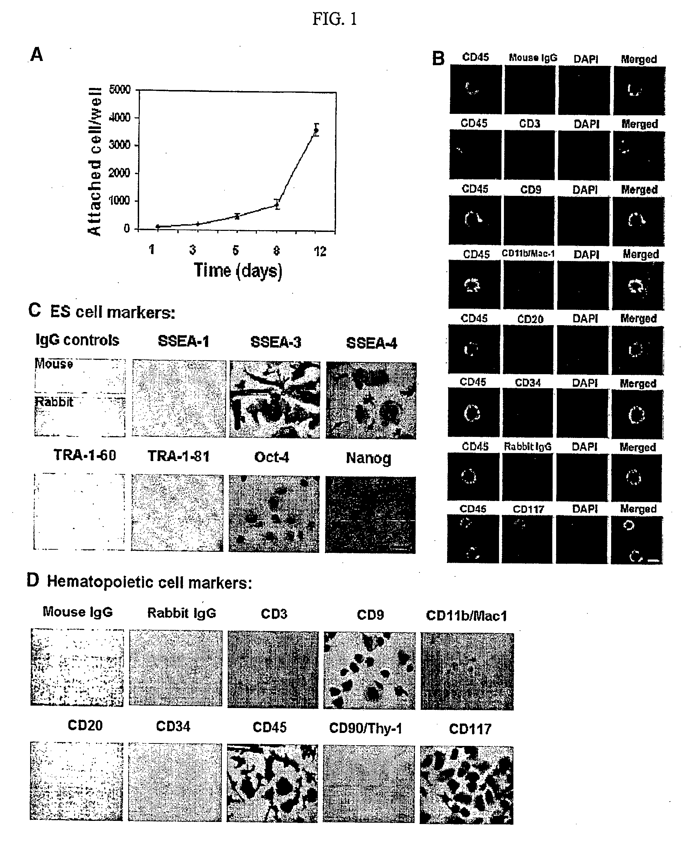

FIG. 1 shows the results of characterization of CB-SC for embryonic and hematopoietic cell markers. (A) Quantitation of attached cells per well over 12 days; (B) Cells attached overnight were stained with antibodies to leukocyte common antigen CD45 and other hematopoietic cell markers, along with DAPI staining; (C) Expression of ES cell markers on 15-day CB-SC; (D) Expression of hematopoietic cell markers on 15-day CB-SC;

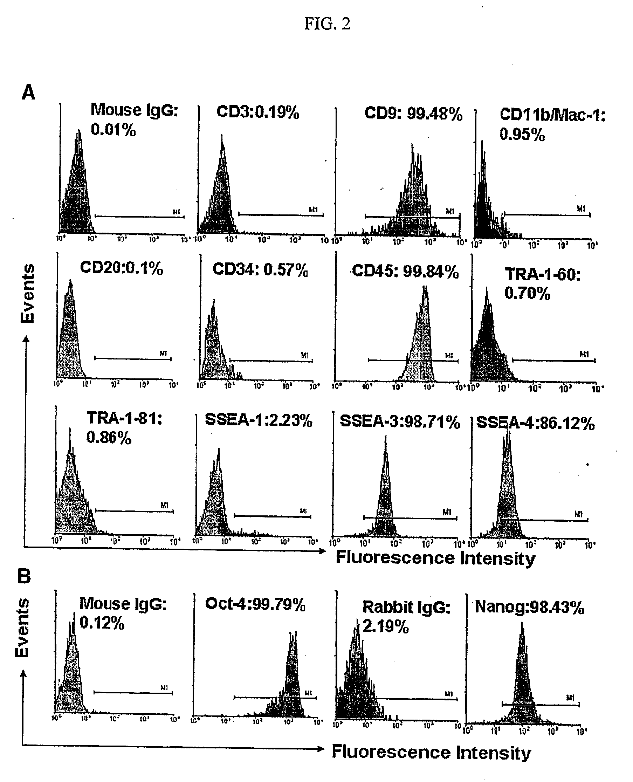

FIG. 2 is the result of flow analyses of embryonic and hematopoietic cell markers on CB-SC (A) and transcription factors (B);

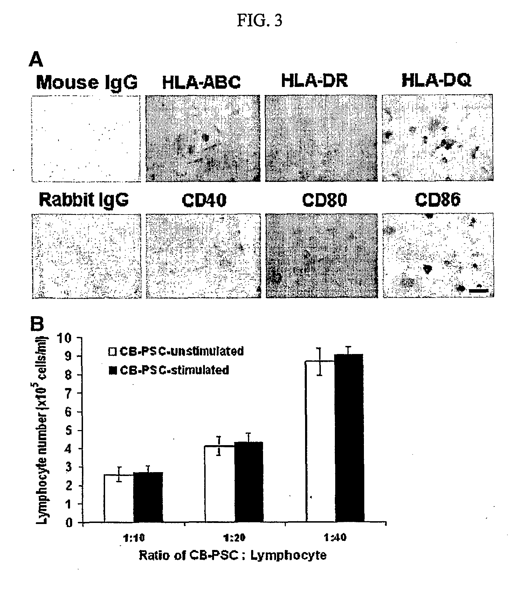

FIG. 3 is the evaluation of the immunogenicity of CB-SC. (A) Examination of immune-associated markers on CB-SC. Immunostaining results were obtained from four cord blood preparations and yielded the similar results. Normal rabbit IgG served as negative for HLA-ABC polyclonal antibody; Isotype-matched mouse IgG antibody served as negative control for other monoclonal antibodies. Scale bar, 47µm. (B) Mixed lymphocyte reaction (MLR). CB-SC were cocultured with allogeneic lymphocytes from human peripheral blood for 6 days at different ratios. Cell number represents mean (± SD) of three experiments;

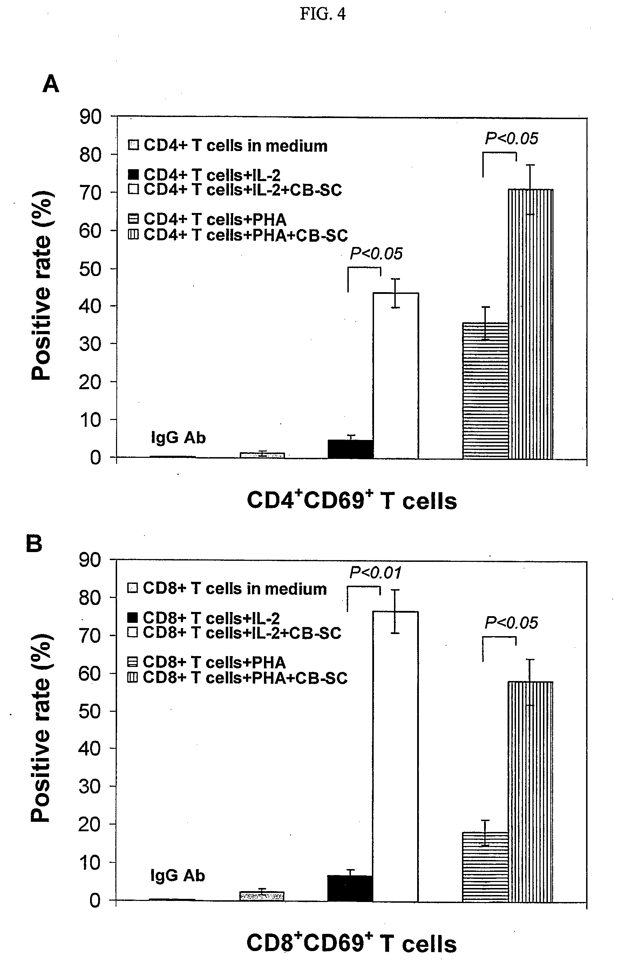

FIG. 4 is the regulation of CB-SC on CD69 molecule expressed by the sorted CD4⁺ and CD8⁺ T cells. The sorted CD4⁺ (*A*) and CD8⁺ (*B*) T cells were cocultured with CB-SC in the presence of IL-2 (500U/ml) and PHA (10µg/ml) for 5 days. CD69 expression was analyzed by flow analysis. Data represent mean (± SD) of three experiments;

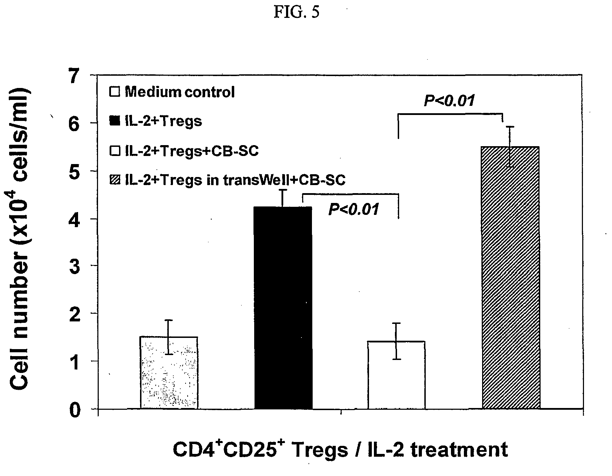

FIG. 5 shows the inhibitory effects of CB-SC on the sorted CD4⁺CD25⁺ regulatory T cells. CD4⁺CD25⁺ regulatory T cells were sorted from allogeneic peripheral blood and cocultured with CB-SC for 5 days, in the presence of interleukin (IL)-2 500U/ml. For cell sorting, cells were double immunostained with FITC-conjugated mouse anti-human CD4 and allophycocyanin (APC)-conjugated mouse anti-human CD25. CD4⁺CD25⁺ regulatory T cells were presented in transwell system as control and evaluate the action of cell-cell contacting on inhibition. Data represent one of at least three experiments with the similar results;

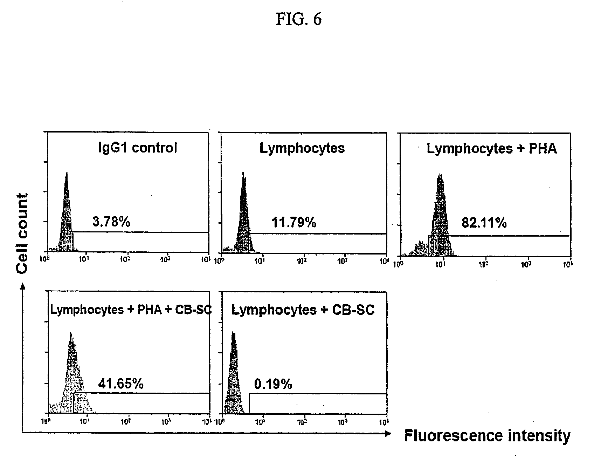

FIG. 6 shows the result of flow analysis of intracellular IL-10 production. Allogeneic lymphocytes were cocultured with CB-SC at ratio 1 : 10 of CB-SC: lymphocytes, in the presence or absence of mitogen PHA. After coculture for 5-6 days, cells were permeablized and evaluated for intracytoplasmic IL-10 level by flow cytometry. Isotype-matched mouse IgG1κ antibody served as negative control. Data represent one of at least three experiments with the similar results;

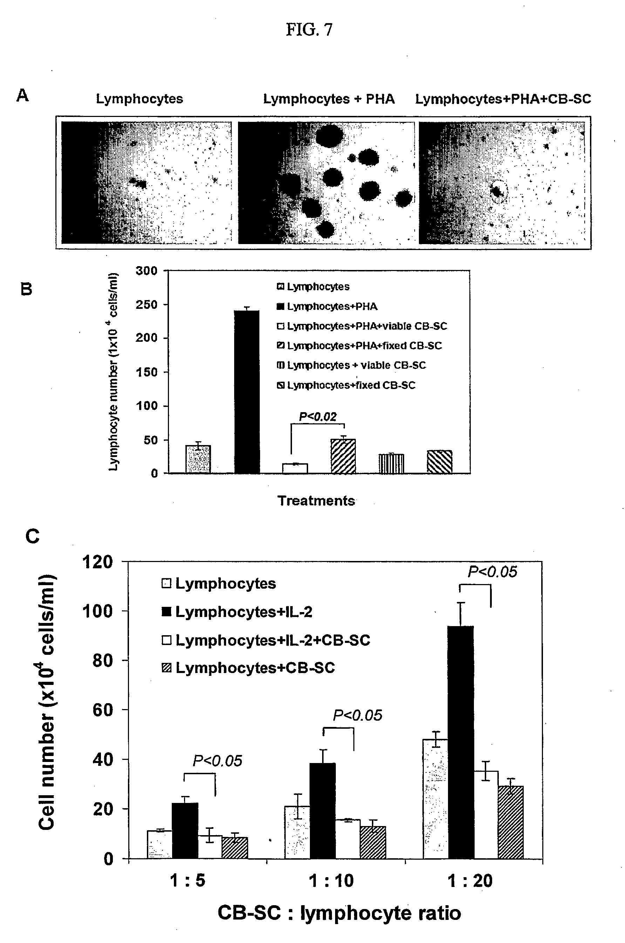

FIG. 7 shows the inhibitory effects of CB-SC on the PHA-stimulated lymphocyte proliferation. *A*, Cell clump formation by phase contrast microscope. PHA-stimulated lymphocytes formed larger cell clumps (middle panel); CB-SC (attached cells) cocultured with PHA-stimulated lymphocytes formed smaller cell clump (marked in red circle). Original magnification, ×50. *B*, Quantification of lymphocyte number. Allogeneic lymphocytes were cocultured with CB-SC for 5-6 days at ratio 1 : 10 of CB-SC: lymphocytes, in the presence or absence of mitogen PHA. *C*, CB-SC were cocultured with allogeneic lymphocytes at different ratios in the presence or absence of 500 U/ml IL-2. Data represent mean (± SD) of four experiments;

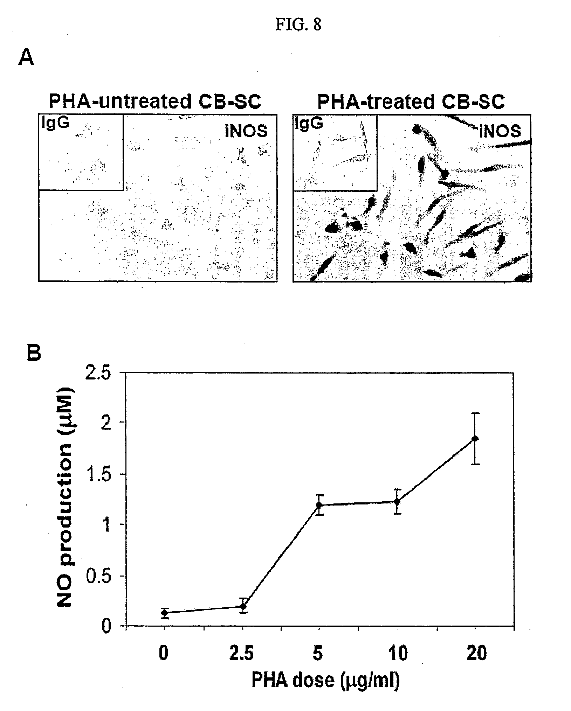

FIG. 8 shows the result of the examination of NO production and iNOS expression. *A*, Immunocytochemistry for iNOS expression in the PHA-treated CB-SC. Normal rabbit IgG (insert, top left) served as negative control for rabbit anti-iNOS polyclonal antibody. Images represent from 10µg/ml PHA-stimulated CB-SC. Immunostaining results were obtained from three cord blood preparations and yielded the similar results. Scale bar, 47µm. *B*, Assay for NO production. CB-SC were treated with PHA at different doses for 3-5 days. The supernatants were collected for NO examination using Griess reaction. Results represent mean (± SD) of three experiments;

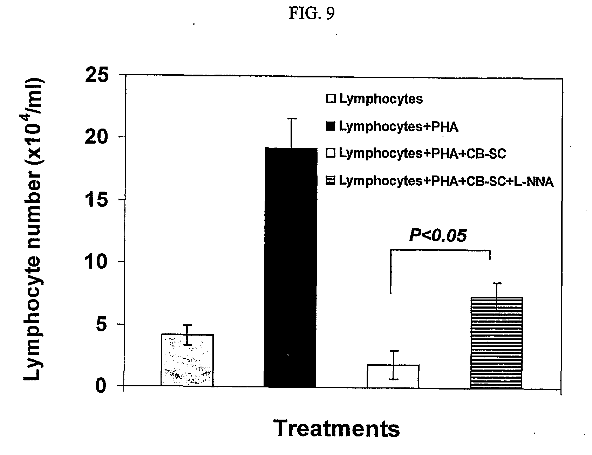

FIG. 9 shows the blocking effects of iNOS inhibitor L-NNA on the lymphocyte proliferation inhibited by coculture with CB-SC. PHA-stimulated lymphocytes were cocultured with CB-SC in the presence or absence of the iNOS inhibitor L-NNA (200µM/day for 3 days). After coculture for 4 days, lymphocytes were harvested for cell count. Cell number represents mean (± SD) of three experiments;

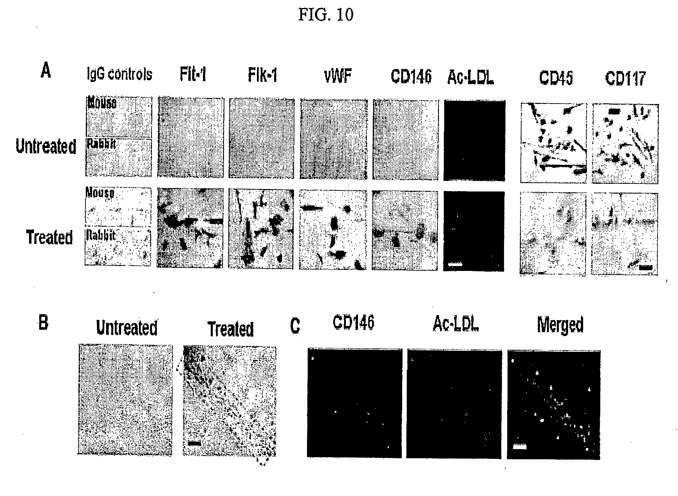

FIG. 10 shows the differentiation of CB-SC into endothelial-like cells. CB-SC were treated with 50ng/ml VEGF for 10-14 days and then prepared for immunostaining. Untreated cells served as control. (A) VEGF-treated or untreated CB-SC were stained with endothelial cell markers Flt-1, Flk-1, von Willebrand Factor (vWF), and CD146 (*left panel*); also evaluated with hematopoietic cell markers CD45 and CD117 (*right panel*). Scale bar, 60µm. (B) Phase contrast image showed formation of cell chain-like structure in VEGF-treated CB-SC. Untreated cells served as control. Scale bar, 50µm. (C) Cells in chain-like structure were double stained with the acetylated low density lipoprotein (Ac-LDL) and CD146 and then merged. The merged image showed overlap of CD146 and Ac-LDL staining. Cells were photographed with a MicroMAX 5MHz Digital Camera using Zeiss Axiovert 100TV Fluorescence microscope. Scale bar, 50µm. The images are representative of five experiments;

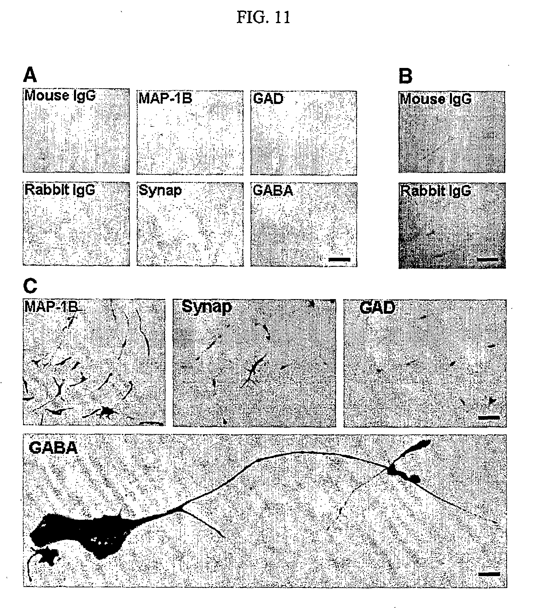

FIG. 11 shows the differentiation of CB-SC into neuronal-like cells. CB-SC were treated with 200ng/ml NGF for 10-14 days and then prepared for immunostaining. Untreated cells served as control. (A) NGF-untreated cells were stained with neuronal markers. Scale bar, 57µm. (B) and (C) showed NGF-treated cells. (B) Mouse IgG served as negative control for microtubule-associated protein-1b (MAP-1b) and synaptophysin (Synap); rabbit IgG served as negative control for γ-aminobutyric acid (GABA) and glutamate decarboxylase_{65/67} (GAD). Scale bar, 57µm. (C) Expression of neuronal cell-specific markers on NGF-treated cells. Scale bar is 37µm. The images are representative of three experiments;

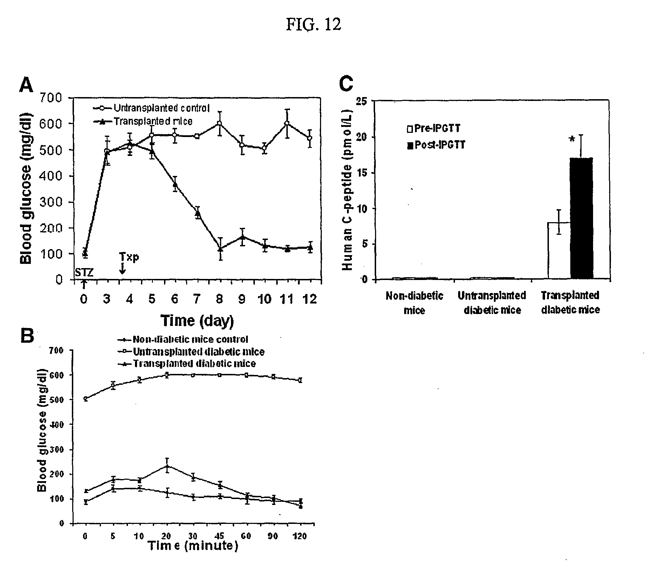

FIG. 12 shows that CB-SC differentiated into functional insulin-producing cells after transplantation into streptozotocin (STZ)-induced diabetic mice. (A) Kinetic examination showed decreasing of blood glucose levels post transplantation (Txp). *N*=7 for CB-SC-transplanted mice; *n*=8 *for* untransplanted diabetic mice. Glucose levels were measured from whole tail vein blood using an AccuChek glucose detector. (B) Intraperitoneal glucose tolerance testing (IPGTT) after 7 days following transplantation. *N*=3 for CB-SC-transplanted mice; *n*=*4 for* untransplanted diabetic mice, and *n*=*3 for* non-diabetic mice. (C) Human C-peptide detection in the sera of transplanted mice. Blood samples were collected during 20 min before and after IPGTT. *N*=3 for each group. Human C-peptide was examined by using an ultrasensitive human C-peptide enzyme-linked immunosorbent assay (ELISA) kit. Data represent mean (± SD). Asteristic (*) represents for *p* value < 0.05;

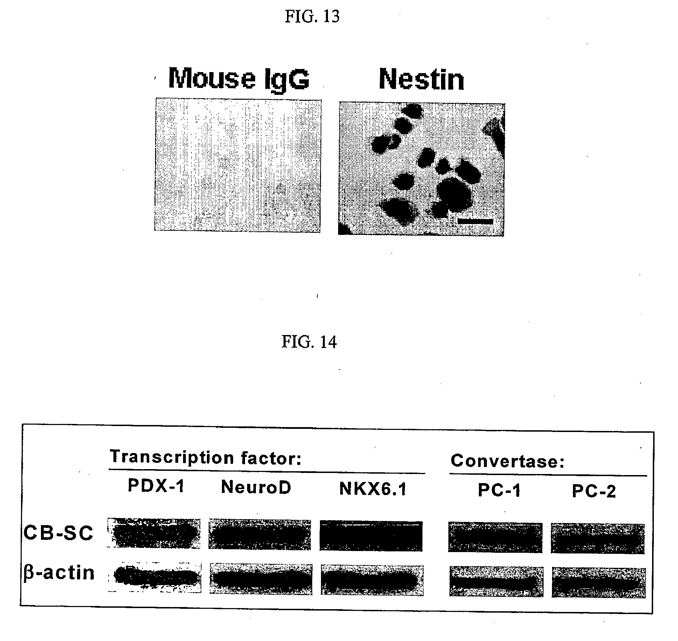

FIG. 13 shows the expression of nestin on CB-SC. Immunostaining of CB-SC with human nestin monoclonal antibody. Isotype-matched IgG1κ served as negative control;
FIG. 14 is the result of Western blot for transcription factors including PDX-1, NeuroD, and NKX6.1, along with prohormone convertases PC-1 and PC-2. β-actin served as internal control;

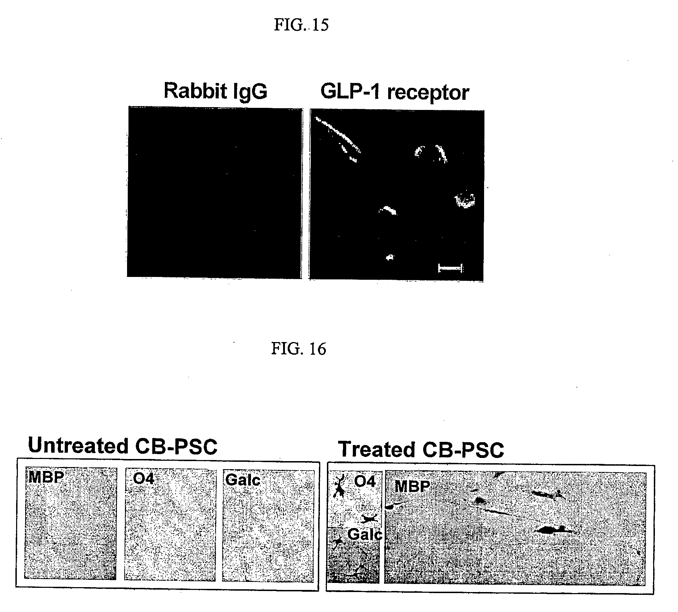

FIG. 15 shows the result of immunostaining for GLP-1receptor on CB-SC. Cells were cultured in 8-well Lab-Teck II chamber slides in regular culture medium and used for immunostaining with rabbit anti-human GLP-1 receptor polyclonal antibody. Normal rabbit IgG served as control for immunostaining. Scale bar, 20µm. Cells were photographed using Zeiss LSM 510 confocal microscope;
FIG. 16 shows the differentiation of CB-SC into the oligodendrocyte. CB-SC were treated with 200ng/ml NGF for 10-14 days. Expression of oligodendrocyte-specific markers on NGF-treated CB-PSC, including myelin basic protein (MBP) (67-74), sulfatide 04, and galactocerebroside (Galc). Original magnification: x400;

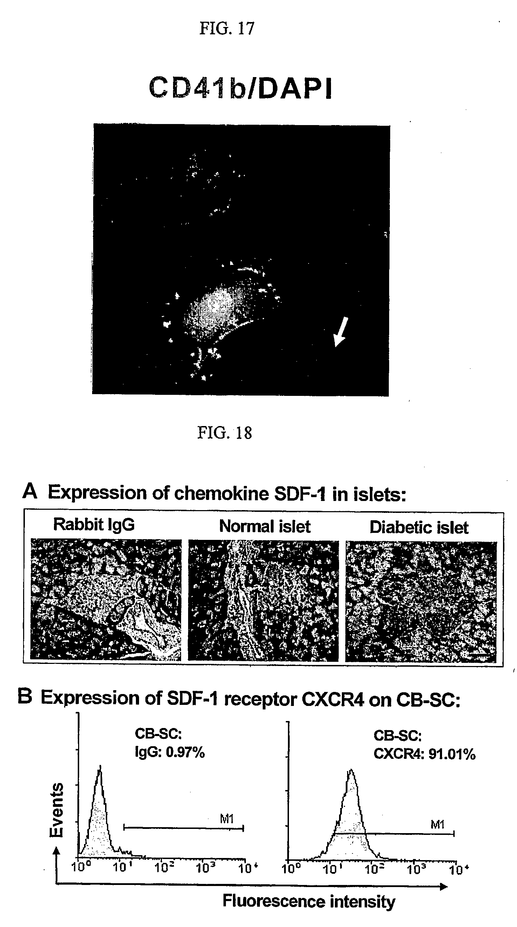

FIG. 17 shows the differentiation of CB-SC into the megakaryocyte. CB-SC were treated with 10ng/ml TPO for 10-14 days. Immunostaining showed expression of megakaryocyte-specific marker CD41b on differentiated CB-SC. DAPI staining showed polyploidy nuclear (grey arrow) and an undifferentiated cell with regular size of nuclear (white arrow). Original magnification: x400; and
FIG. 18 shows the expression of SDF-1 in diabetic islets (A) and its receptor CXCR4 on CB-SC (B). Data represent one of three experiments with the similar results. Scale bar, 36µm.

### DETAILED DESCRIPTION OF THE INVENTION

While this invention is susceptible of embodiment in many different forms, there is shown in the drawings, and will be described herein in detail, specific embodiments thereof with the understanding that the examples are an exemplification of the principles of the invention and are not intended to limit the invention to the specific embodiments illustrated.

The isolated embryonic-like stem cells for use in stem cell-based therapy according to the present invention are isolated from human umbilical cord blood. These stem cells, designated herein as cord blood-stem cells. (CB-SC), represent a unique cell population displaying both embryonic and hematopoietic cell characteristics. CB-SC are characterized as defined in the appended claims.

The present invention discloses a method for isolating the embryonic-like stem cells as defined in the appended claims. The attached cell population can be detached from the culture vessel by, for example, incubation in lidocaine hydrochloride solution wherein the lidocaine hydrochloride is from about 0.1% to about 5%. Optionally, the attached cells can be detached by further incubating the cells with EDTA solution or EDTA solution containing trypsin (trypsin/EDTA) wherein the EDTA is from about 0.5 mM to about 2.5 mM, and the trypsin is from about 0.05% to about 0.25%. Furthermore, the cell culture does not require a cell feeder. What is meant by "non-tissue culture treated culture vessel" as used herein is that the culture vessel has not been treated with vacuum gas plasma prior to cell culture. Examples of suitable culture vessels include, but are not limited to, chamber glass slides and Petri dishes.

As used herein, the term "embryonic stem cell" refers to a stem cell that is derived from the inner cell mass of a blastocyst (e.g., a 4- to 7-day old human embryo) and that is pluripotent. The term "embryonic-like stem cell" refers to a stem cell that is *not* derived from the inner cell mass of a blastocyst.

As used herein, the term "pluripotential", "pluripotential for differentiation" or "pluripotent" refers that the cell is positive for one or more of the pluripotent markers such as but are not limited to Oct-4 and Nanog and the cell has the potential to differentiate to any of the subset of the mammalian body's approximately 260 cell types upon appropriate stimulations such as by the appropriate growth factors.

### Isolation of cord blood-stem cells from human umbilical cord blood

Umbilical cord blood has provided an important source of stem cells for research as it has unique advantages compared to other sources of stem cells: no ethical concerns, no risk to the donors, and low risk of graft-versus-host disease (GVHD) [K. K. Ballen, New trends in umbilical cord blood transplantation, Blood 105 (2005) 3786-3792; P. R. Sanberg, A. E. Willing, S. Garbuzova-Davis, S. Saporta, G. Liu, C.D. Sanberg, P. C. Bickford, S. K. Klasko, N. El-Badri, Umbilical cord blood-derived stem cells and brain repair, Ann N Y Acad Sci. 1049 (2005) 67-83; D. A. Peterson, Umbilical cord blood cells and brain stroke injury: bringing in fresh blood to address an old problem, J Clin Invest. 114 (2004) 312-314]. The present invention involves embryonic-like stem cells isolated from embryonic cord blood. They are designated herein as cord blood-stem cells (CB-SC). As used herein, the terms "umbilical cord blood" and "cord blood" are interchangeable.

CB-SC represent the attached population of cells obtained from culturing the mononuclear cells of the umbilical cord blood after the removal of the red blood cells. These cells are generated using a very basic cell culture medium with a low percentage of serum (e.g., 7% fetal bovine serum), and without cell feeders. This is in contrast to ES cells generated using cell feeders. The requirement of cell feeders for such cells raises potential contamination problems [M. Richards, C. Y. Fong, W. K. Chan, P. C. Wong, A. Bongso, Human feeders support prolonged undifferentiated growth of human inner cell masses and embryonic stem cells, Nat Biotechnol. 20 (2002) 933-936]. An exemplified method of obtaining the CB-SC is shown in Example 1 below. The attached cell population from culturing the mononuclear cells in this Example represented approximately 5% of the mononuclear cells. The cells can be cultured in a culture vessel that is non-tissue culture treated (i.e., no vacuum gas plasma-treated). Examples of such a culture vessel include but are not limited to chamber glass slides and Petri dishes. The cells cannot be cultured in a culture dish which has been vacuum gas plasma-treated. The attached cell population can be detached from the culture vessel by, for example, incubation in lidocaine hydrochloride solution wherein the lidocaine hydrochloride is from about 0.1% to about 5%. Optionally, the attached cells can be detached by further incubating the cells with EDTA solution or EDTA solution containing trypsin (trypsin/EDTA) wherein the EDTA is from about 0.5 mM to about 2.5 mM, and the trypsin is from about 0.05% to about 0.25%. These cells are found to be able to proliferate with an estimated doubling time of 2.8 days based on a growth curve generated over 12 days (FIG. 1A). Using the growth conditions in Example 1, CB-SC can be passaged up to a total of 7 passages over 2 months with the cells passaged every 5 to 7 days. Proliferation decreased after 3 months in culture. However, it is very likely that further optimization of growth conditions with growth factors may improve their potential for longer term proliferation. Examples of these growth factors include but are not limited to leukemia inhibitory factor (LIF), basic frbroblast growth factor (bFGF), interleukin 3, thrombopoietin (TPO), insulin, transferin, all trans-retinoic acid, vitamin D, vitamins, activins, and different concentration of serums.

As compared to other reported cord blood-derived stem cells [K. Bieback, S. Kern, H. Kluter, H. Eichler, Critical parameters for the isolation of mesenchymal stem cells from umbilical cord blood, Stem Cells 22 (2004) 625-634; E. J. Gang, S.H. Hong, J: A. Jeong, S. H. Hwang, S. W. Kim, I. H. Yang, C. Ahn, H. Han, H. Kim, In vitro mesengenic potential of human umbilical cord blood-derived mesenchymal stem cells, Biochem Biophys Res Commun. 321 (2004) 102-108; G. Kogler, S. Sensken, J. A. Airey, T. Trapp, M. Muschen, N. Feldhahn, S. Liedtke, R. V. Sorg, J. Fischer, C. Rosenbaum, S. Greschat, A. Knipper, J. Bender, O. Degistirici, J. Gao, A. I. Caplan, E. J. Colletti, G. Almeida-Porada, H. W. Muller, E. Zanjani, P. Wernet, A new human somatic stem cell from placental cord blood with intrinsic pluripotent differentiation potential, J Exp Med. 200 (2004) 123; M. Aoki, M. Yasutake, T. Murohara, Derivation of functional endothelial progenitor cells from human umbilical cord blood mononuclear cells isolated by a novel cell filtration device, Stem Cells 22 (2004) 994-1002; D. A. Ingram, L.E. Mead, H. Tanaka, V. Meade, A. Fenoglio, K. Mortell, K. Pollok, M.J. Ferkowicz, D. Gilley, M.C. Yoder, Identification of a novel hierarchy of endothelial progenitor cells using human peripheral and umbilical cord blood, Blood 104 (2004) 2752-2760; N. Baal, K. Reisinger, H. Jahr, R. M. Bohle, O. Liang, K. Munstedt, C. V. Rao, K. T. Preissner, M. T. Zygmunt, Expression of transcription factor Oct-4 and other embryonic genes in CD133 positive cells from human umbilical cord blood, Thromb Haemost. 92 (2004) 767-775; M. Yu, Z. Xiao, L. Shen, L. Li, Mid-trimester fetal blood-derived adherent cells share characteristics similar to mesenchymal stem cells but full-term umbilical cord blood does not, Br J Haematol. 124 (2004) 666-675; F. M. Cicuttini, K. Welch, A. W. Boyd, Characterization of CD34+HLA-DR-CD38+ and CD34+HLA-DR-CD38- progenitor cells from human umbilical cord blood, Growth Factors 10 (1994) 127-134], CB-SC are different by displaying the following characteristics: retention of hematopoietic cell antigen CD45 in long-term culture and expression of both ES cell and hematopoietic cell markers. Additionally, both immunocytochemistry and flow analysis demonstrated that CB-SC are negative for CD34 and macrophage marker CD11b/Mac-1, which is significantly different from the previously reported cord blood monocyte-derived stem cells, f-macrophage [Y. Zhao, T. Mazzone, Human umbilical cord blood-derived f-macrophages retain pluripotentiality after thrombopoietin expansion, Exp Cell Res. 310 (2005) 311-318].

McGuckin et al. isolated SSEA-3⁺, SSEA-4⁺, and Oct-4⁺ ES cell-like cells from a non-hematopoietic cell population (CD45⁻) of cord blood [C. P. McGuckin, N. Forraz, M. O. Baradez, S. Navran, J. Zhao, R. Urban, R. Tilton, L. Denner, Production of stem cells with embryonic characteristics from human umbilical cord blood, Cell Prolif. 38 (2005) 245-55]. Compared with CB-SC, these cells grew in clumps and did not spread out in microflasks. Importantly, the rarity of this cell in cord blood (about 0.21% of mononuclear cells [C. P. McGuckin, N. Forraz, M. O. Baradez, S. Navran, J. Zhao, R. Urban, R. Tilton, L. Denner, Production of stem cells with embryonic characteristics from human umbilical cord blood, Cell Prolif. 38 (2005) 245-55] poses a key limitation for therapeutic usefulness.

What is meant by "isolated" in the present invention is that the CB-SC are separated from other cells, such as the red blood cells and other unattached mononuclear cells, found in the umbilical cord blood through one or more isolation methods such as, but are not limited to, mechanical separation or selective culturing. The "isolated" CB-SC population does not have to be pure. Other cell types may be present. The other cell types present may be totally different from CB-SC, or they may be transformed from CB-SC during the cell culture and subsequent passage of the cells. In a preferred embodiment, the isolated population is made up of greater than 50% CB-SC. In yet another preferred embodiment, the isolated population is made up of greater than 75% CB-SC. In a further preferred embodiment, the isolated population is made up of greater than 90% CB-SC.

### CB-SC displaying embryonic stem (ES) cell characteristics

CB-SC in the present invention displays embryonic stem (ES) cell characteristics. What is meant by "embryonic stem cell characteristics" in the present invention is that the stem cells express two critical transcription factors, Oct-4 and Nanog, which are related to the self-renewal and pluripotentiality of ES cells [S. H. Orkin, Chipping away at the Embryonic Stem Cell Network, Cell 122 (2005) 828-830]. In a preferred embodiment, markers characteristic of embryonic stem cell also include other markers such as but are not limited to the stage-specific embryonic antigen SSEA-3 and SSEA-4 [I. Klimanskaya, Y. Chung, L. Meisner, J. Johnson, M. D. West; R. Lanza, Human embryonic stem cells derived without feeder cells, Lancet 365 (2005) 1636-1641]. In yet another preferred embodiment, the "embryonic stem cell characteristics" may further include the weak expression of tumor rejection antigens such as but are not limited to TRA-1-60 and TRA-1-81. In a further embodiment, the "embryonic stem cell characteristics" may further include no expression of SSEA-1.

Immunostaining results (FIG. 1C) showed strong expression of ES cell-specific markers by CB-SC, including the two critical transcription factors Oct-4 and Nanog (related to self-renewal and pluripotentiality of ES cells [S. H. Orkin, Chipping away at the Embryonic Stem Cell Network, Cell 122 (2005) 828-830]), along with stage-specific embryonic antigen (SSEA)-3 and SSEA-4 [I. Klimanskaya, Y. Chung, L. Meisner, J. Johnson, M. D. West, R. Lanza, Human embryonic stem cells derived without feeder cells, Lancet 365 (2005) 1636-1641]. These cells also showed weak expression of tumor rejection antigen TRA-1-60 and TRA-1-81, and no expression of SSEA-1.

### CB-SC displaying hematopoietic cell characteristics

CB-SC in the present invention displays hematopoeitic characteristics, which herein is defined as being positive for the leukocyte common antigen CD45 (CD45 positive, or CD45⁺). Other markers that also indicate displaying of hematopoietic cell characteristics may include markers such as, but are not limited to, tetraspanin CD9 and stem cell factor receptor CD 117.

Immunostaining CB-SC on day 1 after isolation demonstrated the presence of hematopoietic cell antigens including tetraspanin CD9, leukocyte common antigen CD45, and stem cell factor receptor CD117 (FIG. 1B). The strong expression of CD45 by CB-SC is an indication that these cells are of hematopoietic origin, which are different from many other adult stem cells which are of mesenchymal origin and are CD45 negative (CD45⁻).

### CB-SC phenotypically different from hematopoietic stem cells

CB-SC are phenotypically different from hematopoeitic stem cells. Hematopoeitic stem cells are characterized by being positive for the CD34 marker (CD34⁺). Immunostaining studies indicated that CB-SC are CD34 negative (CD34⁻) (FIB. 1B), and, therefore, are phenotypically different from hematopoeitic stem cells.

### CB-SC phenotyptically different from lymphocytes, macrophages and monocytes

These cells do not express the macrophage marker CD11b/Mac-1, T lymphocyte marker CD3, or B lymphocyte marker CD20 (FIG. 1B). These results indicate that CB-SC are phenotypically distinct from lymphocytes, macrophages and monocytes, and CB-SC are not monocyte-derived.

### CB-SC displaying low immunogenicity and immune regulation

A major concern using stem cells for therapeutics is their immunogenicity, leading to immune rejection. Cellular immunogenicity is mainly determined by the major histocompatibility complex (MHC) including MHC class I molecule (HLA-ABC) and MHC Class II molecules (HLA-DR and HLA-DQ) [Drukker, G. Katz, A. Urbach, M. Schuldiner, G. Markel, J. Itskovitz-Eldor, B. Reubinoff, O. Mandelboim, N. Benvenisty, Characterization of the expression of MHC proteins in human embryonic stem cells, Proc Natl Acad Sci USA. 99(2002) 9864-9869]. As shown in Example 4 below, immunostaining for two critical MHC molecules demonstrated that only 5% of cells expressed HLA-ABC, and HLA-DR was completely negative (FIG. 3A). These levels of expression are similar to levels of expression reported in human ES cells [Drukker, G. Katz, A. Urbach, M. Schuldiner, G. Markel, J. Itskovitz-Eldor, B. Reubinoff, O. Mandelboim, N. Benvenisty, Characterization of the expression of MHC proteins in human embryonic stem cells, Proc Natl Acad Sci USA. 99(2002) 9864-9869]. Approximately 6% of cells showed weak expression for CD40 and CD80; ≈30% of cells expressed HLA-DQ; ≈22% of cells expressed CD86 (FIG. 3A).

Low immunogenicity of CB-SC can also be readily demonstrated with a functional analysis using the mixed lymphocyte reaction as described in Exmaple 5 below. As shown in FIG. 3B, CB-SC did not stimulate lymphocyte proliferation in an allogeneic mixed lymphocyte reaction, consistent with low immunogenicity demonstrated by immunostaining.

Not to be bound by any specific theory, the low immunogenicity of CB-SC may be contributed to the ability of CB-SC to regulate T-lymphocytes. As shown in Example 7 below, CB-SC, when cocultured with allogeneic peripheral blood lymphocytes in the presence of the mitogen phytohaemagglutinin (PHA) or physiological growth factor interleukin (IL)-2, decreases the percentage of PHA-stimulated CD8⁺ T cells and IL-2-stimulated CD4⁺CD25⁺ regulatory T cells, along with normalization of the CD4/CD8 ratio and decreasing of intracellular IL-10 level. CD69 molecule, a negative regulator on activated T lymphocytes, was significantly increased on both CD4⁺ and CD8⁺ T lymphocytes after coculture with CB-SC (FIG.4). In addition, CB-SC significantly inhibits the proliferation of IL-2- and/or PHA-stimulated lymphocytes. Mechanism studies showed that nitric oxide (NO) partially mediated this inhibitory effect, as demonstrated by blocking with a powerful nitric oxide synthase inhibitor (N-omega-nitro-L-arginine, L-NNA). Cell-cell contacting play a critical role in IL-2 treatment, as demonstrated by using transwell culture system (FIG.5).

### CB-SC have the capability for proliferation

One of the key characteristics for a stem cell to be suitable for stem cell-based therapy is its capability for proliferation. As used herein, the term "capability for proliferation" refers that the cell expresses one or more self-renewal markers such as but are not limited to Nanog and the cell can proliferate. Preferably, the cell can proliferate indefinitely. What is meant by "proliferate" as used in the present disclosure is that the cell can grow and multiply in numbers when the cell is cultured. The terms "proliferate" and "expand" are used interchangeably herein.

As indicated by immunostaining studies (FIG. 1C), CB-SC are positive for the critical transcription factor Nanog related to self-renewal of ES cells. These cells are found to be able to proliferate with an estimated doubling time of 2.8 days based on a growth curve generated over 12 days (FIG. 1A). Using the growth conditions in Example 1 shown below, CB-SC can be passaged up to a total of 7 passages over 2 months with the cells passaged every 5 to 7 days. However, it is likely that further optimization of growth conditions may improve their potential for longer term proliferation.

### CB-SC have the ability to differentiate to multiple types of cells

Another key characteristic for a stem cell to be suitable for stem-cell therapy has the ability to differentiate to multiple types of cells. Preferably, the stem cell is pluripotent. Our data indicate that CB-SC, upon appropriate stimulations, can differentiate into a wide variety of types of cells with characteristics of three embryonic layers (mesoderm, ectoderm and endoderm), which include endothelial-like cells (which are mesoderm-derived [L. M. Hoffman, M.K. Carpenter, Characterization and culture of human embryonic stem cells, Nat Biotechnol. 23 (2005) 699-708; M. Baron, Induction of embryonic hematopoietic and endothelial stem/progenitor cells by hedgehog-mediated signals, Differentiation 68 (2001) 175-185]), neuronal-like cells (which are ectoderm-derived [L. M. Hoffman, M.K. Carpenter, Characterization and culture of human embryonic stem cells, Nat Biotechnol. 23 (2005) 699-708; L. Bally-Cuif, M. Hammerschmidt, Hammerschmidt. Induction and patterning of neuronal development, and its connection to cell cycle control, Curr Opin Neurobiol. 13 (2003) 16-25]), and insulin-producing cells (which are endoderm-derived [M. A. Hussain, N. D. Theise, Stem-cell therapy for diabetes mellitus, Lancet 364 (2004) 203-205; L. M. Hoffman, M.K. Carpenter, Characterization and culture of human embryonic stem cells, Nat Biotechnol. 23 (2005) 699-708]). Other cells that CB-SC can differentiate to include oligodendrocytes and megakaryocyte-like cells. It is very likely that CB-SC can differentiate to other types of cells as well.

As shown in Example 4 below (FIG. 1C), CB-SC is positive for both Oct-4 and Nanog which are critical transcription factors related to pluripotency. Therefore, it is reasonable to speculate that CB-SC are pluripotent.

### CB-SC are suitable for stem cell-based-therapies

The present invention provides a composition for stem cell-based therapies comprising CB-SC. Also described is a method for stem cell-based therapies by the use CB-SC.

Embryonic stem (ES) cells display two unique properties: capability to proliferate and pluripotentiality for differentiation [A. Vats, R. C. Bielby, N. S. Tolley, R. Nerem, J.M. Polak, Stem cells, Lancet 366 (2005) 592-602]. Stem cell-based therapy, therefore, has significant potential to cure important, and common, human diseases [M. A. Hussain, N. D. Theise, Stem-cell therapy for diabetes mellitus, Lancet 364 (2004) 203-205; C. M. Rice, N.J. Scolding, Adult stem cells--reprogramming neurological repair? Lancet 364 (2004) 193-199]. However, a major limitation for stem cell-based therapy has been identification of a suitable source of stem cells. For instances, there are significant ethical issues for use of ES cells [C. Dennis, Check E, 'Ethical' routes to stem cells highlight political divide, Nature 437 (2005) 1076-1077; M. Evans, Ethical sourcing of human embryonic stem cells--rational solutions? Nat Rev Mol Cell Biol. 6 (2005) 663-667] and adult stem cells display reduced proliferation and differentiation ability [C. M. Rice, N.J. Scolding, Adult stem cells--reprogramming neurological repair? Lancet 364 (2004) 193-199].

CB-SC share the same key characteristics of embryonic stem cells in capability to proliferate and pluripotentiality for differentiation. Combining with their low immunogenicity, CB-SC are suitable for stem cell-based therapies in treatment of human diseases. In addition, CB-SC are readily available from umbilical cord blood, and they can be cultured and propagated *in vitro* to provide an abundant supply of cells for stem cell-based therapies. These properties of CB-SC can overcome the problem of inadequate availability and supply associated with ES cells and other adult stem cells displaying reduced proliferation and differentiation ability.

### Use of CB-SC for treating hyperglycemia in diabetic subjects

Described herein is a method for treating hyperglycemia in a diabetic mammalian subject by administering CB-SC to the subject. The administered CB-SC migrate to the pancreas of the subject and differentiate to functional insulin-producing cells *in vivo,* which in turn produce insulin in response to the high, glucose level to control hyperglycemia in the subject.

Diabetes and its long-term complications are increasing in prevalence posing an important therapeutic challenge for individual patients and public health. Deficit of insulin-producing cells is the crucial issue for both type 1 and type 2 diabetic patients. In spite of the development and application of various insulin formulations, exogenous insulin neither achieves the same degree of glycemic control as that provided by endogenous insulin, nor completely prevents the long-term complications such as diabetic retinopathy, neuropathy, nephropathy, and diverse cardiovascular disorders [M. A. Hussain, N. D. Theise, Stem-cell therapy for diabetes mellitus, Lancet 364 (2004) 203-205]. These clinical challenges necessitate the development of more efficient treatments. Islet cell transplantation, a potential treatment, has been limited by a shortage of pancreas as a source of purified islets. Stem cell-derived insulin-producing cells, therefore, provide a promising approach for beta cell-replacement therapy [M. A. Hussain, N. D. Theise, Stem-cell therapy for diabetes mellitus, Lancet 364 (2004) 203-205]. Accumulating evidence suggests that insulin-producing cells derived from stem cells can normalize blood glucose in diabetic animal models [M. A. Hussain, N. D. Theise, Stem-cell therapy for diabetes mellitus, Lancet 364 (2004) 203-205]. However, in previous reports these cells were derived from ES cells and fetal tissues [G. K. Brolen, N. Heins, J. Edsbagge, H. Semb, Signals from the embryonic mouse pancreas induce differentiation of human embryonic stem cells into insulin-producing beta-cell-like cells, Diabetes 54 (2005) 2867-2874; Y. Hori, I. C. Rulifson, B. C. Tsai, J. J. Heit, J. D. Cahoy, S. K. Kim, Growth inhibitors promote differentiation of insulin-producing tissue from embryonic stem cells, Proc Natl Acad Sci USA. 99 (2002) 16105-16110; H. Segev, B. Fishman, A. Ziskind, M. Shulman, J. Itskovitz-Eldor, Differentiation of human embryonic stem cells into insulin-producing clusters, Stem Cells 22 (2004) 265-274; S. Miyazaki, E. Yamato, J. Miyazaki, Regulated expression of pdx-1 promotes in vitro differentiation of insulin-producing cells from embryonic stem cells, Diabetes 53(2004) 1030-1037; M. Zalzman, L. Anker-Kitai, S. Efrat, Differentiation of human liver-derived, insulin-producing cells toward the beta-cell phenotype, Diabetes 54(2005) 2568-2575; M. Zalzman, S. Gupta, R. K. Giri, I. Berkovich, B. S. Sappal, O. Karnieli, M. A. Zern, N. Fleischer, S. Efrat, Reversal of hyperglycemia in mice by using human expandable insulin-producing cells differentiated from fetal liver progenitor cells, Proc Natl Acad Sci USA. 100(2003)7253-7258], raising ethical concerns for their clinical application. CB-SC can correct hyperglycemia in diabetic mice, and restore euglycemia after an acute glucose challenge (IPGTT).

As shown in Example 10 below, CB-SC injected into the peritoneal cavity of STZ-induced diabetic mice has the capability to correct hyperglycemia in these mice. Further, intraperitoneal glucose tolerance testing (IPGTT) demonstrated physiological responses of transplanted CB-SC cells. Blood glucose of normal non-diabetic mice peaked between 5-10min and returned to normal level 30min following glucose challenge. Blood glucose of CB-SC-transplanted diabetic mice peaked at 20min, followed by a return to normal range after 60min (FIG. 10B). However, blood glucose of CB-SC-untransplanted diabetic mice remained very high (>500mg/dl) (FIG. 12B).

To substantiate that this reversal of hyperglycemia was associated with differentiation of CB-SC into insulin-producing cells, we also examined the production of human C-peptide (as an indicator of human insulin secretion) in the sera of CB-SC-transplanted diabetic mice. Human C-peptide production was undetectable in the sera of non-diabetic mice and untransplanted diabetic mice, but acutely increased in transplanted mice following glucose challenge. These results, in aggregate, provide evidence that CB-SC give rise to functional insulin-producing cells after transplantation into diabetic mice. This conclusion is supported by a recent report, which showed cord blood may contain progenitors that generate insulin-producing cells [S. Yoshida, F. Ishikawa, N. Kawano, K. Shimoda, S. Nagafuchi, S. Shimoda, M. Yasukawa, T. Kanemaru, H. Ishibashi, L. D. Shultz, M. Harada, Human cord bloodderived cells generate insulin-producing cells in vivo, Stem Cells 23(2005) 1409-1416].

### Examples

Statistical analyses of data in the following examples were performed by the paired Student's t-test to determine statistical significance. Values are given as mean ± SD (standard deviation).

### Example 1: Isolation of cord blood-stem cells (CB-SC) from human umbilical cord blood and cell culture

Human umbilical cord blood samples (50-100 ml/unit) were obtained from healthy donors (Life-Source Blood Services, Glenview, IL). Mononuclear cells were isolated using Ficoll-Hypaque (γ=1.077, Sigma), followed by removing red blood cells using Red Blood Cell Lysis buffer (eBioscience, San Diego, CA). Mononuclear cells were seeded into 8-Well Lab-Tek II Chamber Slides (Fisher Scientific) at 1-2×10⁵cells/ml, 0.5ml/well in RPMI 1640 medium supplemented with 7% fetal bovine serum (Invitrogen, Carlsbad, CA), and incubated at 37°C, 8% CO₂ conditions. Cells at 70-80% confluence were passaged every 5-7 days with the fresh RPMI 1640 medium supplemented with 7% fetal bovine serum at ratio 1:2. To expand cells on a large scale, mononuclear cells were initially seeded in 150x15 mm Petri dishes (Becton Dickinson Labware, Franklin Lakes, NJ) at 1×10⁶cells/ml, 25ml/dish in RPMI 1640 medium supplemented with 7% fetal bovine serum. Cells were passaged every 10-14 days at a ratio of 1:2. Because CB-SC tightly adhered to the culture dishes, they were resistant to routine trypsin-EDTA (0.53mM EDTA), or 5mM EDTA for detachment. We therefore incubated the attached cells in 3.5% lidocaine hydrochloride (Sigma) with 0.5 mM EDTA (diluted from 0.5M EDTA, PH 8.0, Invitrogen Corporation, Carlsbad, CA) for 5-8 minutes at room temperature to harvest cells for in *vitro* analysis and in vivo transplantation. Trypsin/EDTA may be used following lidocaine detachment in order to facilitate cell detachment and keep cell viability.

The attached cell population obtained by culturing the mononuclear cells of cord blood cultured in the 8-well Lab-Tek chamber slides (approximately 5% of mononuclear cells) could proliferate. These cells were therefore passaged every 5-7 days, up to a total of 7 passages over 2 months. Based on a growth curve generated over 12 days (FIG. 1A), we estimated the doubling time of CB-SC to be 2.8 days. Cell proliferation ability decreased in the longer-term cultures (over 3 months).

### Example 2: Immunochemistry

Immunostaining was performed as previously described with minor modifications [Y. Zhao, T. Mazzone, Human umbilical cord blood-derived f-macrophages retain pluripotentiality after thrombopoietin expansion, Exp Cell Res. 310 (2005) 311-318]. The cells were incubated for 20 minutes at room temperature with ImmunoPure Peroxidase Suppressor (Pierce, Rockford, IL) to block endogenous perioxidase activity. For fluorescence-labeled immunostaining, this step was omitted. After incubation with primary antibodies, cells were stained with ABC kit (Vector Laboratories, Burlingame, CA). Immunostaining was performed using the following antibodies: mouse anti-human monoclonal antibodies CD3, CD9, CD11b/Mac-1 (Clone ICRF44), CD20, CD34 (clone 563), R-PE-conjugated mouse anti-human CD34 monoclonal antibody (clone 563), CD45 (HI30), FITC-conjugated mouse anti-human CD45 monoclonal antibody (HI30), CD146 (Clone P1H12), human leukocyte antigen (HLA)-DR, HLA-DQ, isotype-matched antibody IgG₁κ were purchased from BD Pharmingen; mouse monoclonal antibodies SSEA-1, SSEA-3, SSEA-4, TRA-1-60, TRA-1-81, Oct-4, Thy-1 (CD90), synaptophysin, and tyrosine hydroxylase (TH) were from Chemicon International Inc. (Temecula, CA); mouse monoclonal antibodies CD11b and anti-human von Willebrand factor (vWF) were from Sigma (Saint Louis, MO); microtubule-associated protein (MAP) 1B antibody was from NeoMarkers. Rabbit anti-human polyclonal antibodies: Nanog antibody and glutamate decarboxylase_{65/67} antibody were from Chemicon; γ-aminobutyric acid (GABA) antibody was from Sigma; antibodies CD117, Flt-1 (vascular endothelial growth factor receptor 1, VEGF R1), Flk-1 (VEGF R2) were from NeoMarkers. Rabbit anti-human HLA-ABC polyclonal antibody was from Santa Cruz Biotechnology (Santa Cruz, CA). Mouse monoclonal antibodies to human CD40, CD80 (B7-1), and CD86 (B7-2) were from eBioscience (San Diego, CA). Cells were then incubated with primary antibodies, the mouse IgG₁κ (as control for mouse monoclonal antibodies), and normal rabbit IgG (as control for rabbit polyclonal antibodies, Santa Cruz). FITC- or rhodamine (TRITC)-conjugated AffiniPure Donkey antimouse IgG antibodies were obtained from Jackson ImmunoResearch Laboratories, INC. (West Grove PA). Alexa Fluor 568-conjugated second antibody was from Molecular Probes (Carlsbad, CA). After staining, the slides were mounted with Mounting Medium (Vector Laboratories, Burlingame, CA). Cells were viewed and photographed using a Zeiss Axiocam Color Camera with Zeiss Axioskop Histology/Digital Fluorescence microscope. The images were acquired with the manufacturer's software and edited using Adobe Photoshop Elements 2.0. When we evaluated cell percentage of expression of cells antigens, we used mouse or rabbit IgG staining as controls for background staining (<1%). Any cellular staining higher than the background staining, were regarded as positive staining and then quantified. At least 400 cells were evaluated from five randomly selected fields of each slide.

For double staining assay in the differentiation of endothelial-like cells, both VEGF-treated and untreated cells first completed incorporating acetylated low density lipoprotein labeled with 1,1'-diocatadecyl-3,3,3',3'-tetramethylindo-carbocyanine perchlorate (Dil-Ac-LDL, Biomedical Technologies Inc., Stroughton, MA), and then were fixed with 4% formaldehyde for 20 min at room temperature and used for immunostaining with specific cell surface marker CD146 as described above.

### Example 3: Flow analysis

For intracellular staining, cells were fixed with 4% paraformaldehyde for 20 min and then permeabilized with 0.5% Triton X-100 (Sigma) for 5-6 min at room temperature. For cell surface staining, the fixation and permeabilization steps were omitted. Cells were incubated with 2.5% horse serum (Vector Laboratories) at room temperature to block non-specific staining. Cells were incubated with primary antibodies for 45 min at 4°C and then washed with cold PBS. Cells were stained with FITC-conjugated second antibodies for another 45 min at 4°C and followed by flow analysis. Isotype-matched mouse IgG₁κ antibody or normal rabbit IgG served as negative controls. After staining, cells were analyzed using a Beckman-Coulter Elite ESP.

### Example 4: Characterization of CB-SC

Immunostaining CB-SC on day 1 after isolation demonstrated the presence of hematopoietic cell antigens including tetraspanin CD9, leukocyte common antigen CD45, and stem cell factor receptor CD117 (FIG. 1B). These cells did not express the macrophage marker CD11b/Mac-1, T lymphocyte marker CD3, B lymphocyte marker CD20, or the hematopoietic stem cell marker CD34 (FIG. 1B). These results suggest that CB-SC are phenotypically distinct from macrophages, lymphocytes, and previously characterized CD34⁺ hematopoietic stem cells. To further characterize these cells, we evaluated expression of embryonic markers. Immunostaining results showed strong expression of ES cell-specific markers, including two critical transcription factors Oct-4 and Nanog (related to self-renewal and pluripotentiality of ES cells [S. H. Orkin, Chipping away at the Embryonic Stem Cell Network, Cell 122 (2005) 828-830]), along with stage-specific embryonic antigen (SSEA)-3 and SSEA-4 [I. Klimanskaya, Y. Chung, L. Meisner, J. Johnson, M. D. West, R. Lanza, Human embryonic stem cells derived without feeder cells, Lancet 365 (2005) 1636-1641]. These cells also showed weak expression of tumor rejection antigen (TRA)-1-60 and TRA-1-81, and no expression of SSEA-1 (FIG. 1C). Consistent with staining results at the early stage (FIG. 1B), all cells strongly expressed hematopoietic cell markers, including CD9, CD45, and CD117, but remained negative for CD3, CD11b/Mac-1, CD20, CD34, and CD90/Thy-1 surface antigens (FIG. 1D). Notably, flow analysis demonstrated that CB-SC maintained in 7% FBS-RPMI 1640 medium retained these phenotypes for over 2 months (FIG. 2 A and B). The above results were obtained from 8 cord blood units yielding similar results, including fresh and liquid nitrogen-frozen preparations. Based on our current studies, CB-SC can be generated from every cord blood unit. Together, these data indicate CB-SC represent a unique cell population displaying both embryonic and hematopoietic cell characteristics.

### Example 5: Immunogenicity-associated phenotypes of CB-SC

A major concern using stem cells for therapeutics is their immunogenicity, leading to immune rejection. Cellular immunogenicity is mainly determined by the major histocompatibility complex (MHC) including MHC class I molecule (HLA-ABC) and MHC Class II molecules (HLA-DR and HLA-DQ) [Drukker, G. Katz, A. Urbach, M. Schuldiner, G. Markel, J. Itskovitz-Eldor, B. Reubinoff, O. Mandelboim, N. Benvenisty, Characterization of the expression of MHC proteins in human embryonic stem cells, Proc Natl Acad Sci USA. 99(2002) 9864-9869]. To more fully characterize CB-SC, we evaluated CB-SC for immunogenicity-associated phenotypes including HLA-ABC, HLA-DR, and HLA-DQ; along with immune response-related costimulating molecules CD40, CD80, and CD86. Immunostaining for two critical MHC molecules demonstrated that only 5% of cells expressed HLA-ABC, and HLA-DR was completely negative (FIG. 3A). These levels of expression are similar to levels of expression reported in human ES cells [Drukker, G. Katz, A. Urbach, M. Schuldiner, G. Markel, J. Itskovitz-Eldor, B. Reubinoff, O. Mandelboim, N. Benvenisty, Characterization of the expression of MHC proteins in human embryonic stem cells, Proc Natl Acad Sci USA. 99(2002) 9864-9869]. Approximately 6% of cells showed weak expression for CD40 and CD80; ≈30% of cells expressed HLA-DQ; ≈22% of cells expressed CD86 (FIG. 3A).

### Example 6: Effect of CB-SC on stimulating the proliferation of allogeneic lymphocytes

To more fully evaluate their immunogenicity, we performed a functional analysis using the mixed lymphocyte reaction. CB-SC as stimulator were seeded in 8-Well Lab-Tek II Chamber Slides at 1×10⁵cells/ml, 0.5ml/well in RPMI 1640 medium supplemented with 7% fetal bovine serum and incubated at 37°C, 8% CO₂ conditions overnight. Allogenetic lymphocyte as responder were collected from buffy coats of healthy donors (Life-Source Blood Services, Glenview, IL) after removing all attached cells and then cocultured with CB-SC in triplicate at increasing CB-SC : Lymphocyte ratios (1:10, 1:20, and 1:40). Lymphocyte cultures without CB-SC served as controls. After 6 days, the suspended lymphocytes were collected and counted. As shown in FIG. 3B, CB-SC did not stimulate lymphocyte proliferation in an allogeneic mixed lymphocyte reaction, consistent with low immunogenicity demonstrated by immunostaining.

### Example 7: Immune regulation of CB-SC on T-lymphocytes

In order to further understand the relationship between lymphocytes and CB-SC, we studied the immune regulation of CB-SC on T-lymphocytes.

CB-SC adhere very tightly to the culture dishes and display large rounded morphology, it is easy to distinguish between CB-SC and lymphocytes. CB-SC growing at 80% confluence were used for coculture with allogeneic lymphocytes. Allogeneic lymphocytes were collected from buffy coats of healthy donors (Life-Source Blood Services, Glenview, IL) after Ficoll-Hypaque separation followed by removing all attached cells and then cocultured with CB-SC at the ratio (1:10) [Zhao, Y., H. Wang, and T. Mazzone. 2006. Identification of stem cells from human umbilical cord blood with embryonic and hematopoietic characteristics. Exp Cell Res. 2006, 312: 2454-2464. DOI: 10.1016/j.yexcr.2006.04.008. April 26; (Epub ahead of print)] of CB-SC.

For PHA stimulation, lymphocyte suspensions (1×10⁶ cells/ml) with or without PHA (10µg/ml, Sigma) were seeded onto CB-SC cell cultures in regular culture medium. PHA-stimulated lymphocytes without CB-SC served as positive control; lymphocytes cultured only in regular medium served as negative control. Lymphocytes cocultured with CB-SC without PHA stimulation served as an additional negative control. For IL-2 stimulation, IL-2 (500 U/ml, eBioscience) was used. After 5-6 days, the suspended lymphocytes were collected for cell count and/or flow analysis.

To evaluate the effect of cell-cell contact on inhibition of lymphocyte proliferation, CB-SC were fixed with 2.5% glutaraldehyde for 2 hours at room temperature followed by five washes with PBS and then used for coculture experiments as described above. Unfixed CB-SC from same cord blood units served as controls. Additionally, we employed transwell culture system with 0.4µm size (Sigma) to plant lymphocytes.

In experiments where N^{ω} -nitro-L-arginine (L-NNA, Sigma) was used to inhibit NO synthase, the drug (200µM L-NNA) was added 1h before PHA stimulation and then administrated 200µM/day for additional two days. [Ziche, M., L. Morbidelli, E. Masini, S. Amerini, H. J. Granger, C. A. Maggi, P. Geppetti, and F. Ledda. 1994. Nitric oxide mediates angiogenesis in vivo and endothelial cell growth and migration in vitro promoted by substance P. J Clin Invest. 94:2036-2044].

Flow analysis was preformed as previously described [Zhao, Y., H. Wang, and T. Mazzone. 2006. Identification of stem cells from human umbilical cord blood with embryonic and hematopoietic characteristics. Exp Cell Res. 2006, 312: 2454-2464. DOI: 10.1016/j.yexcr.2006.04.008. April 26; (Epub ahead of print)]. In brief, for intracellular IL10 staining, cells were fixed with 4% paraformaldehyde for 20 min and then permeabilized with 0.5% Triton X-100 (Sigma) for 5-6 min at room temperature. Cells were incubated with 2.5% horse serum (Vector Laboratories) at room temperature to block non-specific staining. Cells were incubated with mouse anti-human IL-10 monoclonal antibody (R &D Systems, Minneapolis, MN) for 45 min at 4°C and then washed with cold PBS. Cells were stained with FITC-conjugated second antibody for another 45 min at 4°C and followed by flow analysis. For cell surface staining, the fixation and permeabilization steps were omitted. Cells were incubated with FITC-conjugated mouse anti-human CD4, PE-conjugated mouse anti-human CD8, and allophycocyanin (APC)-conjugated mouse anti-human CD25 (eBioscience, San Diego, CA) for 45 min at 4°C and followed by flow analysis. Isotype-matched mouse IgG₁κ antibody (BD Pharmingen) served as negative control. After staining, cells were analyzed using a CyAn ADP (DakoCytomation).

NO production was determined by using the Griess reagent [Ziche, M., L. Morbidelli, E. Masini, S. Amerini, H. J. Granger, C. A. Maggi, P. Geppetti, and F. Ledda. 1994. Nitric oxide mediates angiogenesis in vivo and endothelial cell growth and migration in vitro promoted by substance P. J Clin Invest. 94:2036-2044] (1% sulfanilamide, 0.1% naphthylenediamine dihydrochloride, 2.5% H3PO4, Sigma) in supernatants of PHA-stimulated and/or -unstimulated CB-SC. CB-SC were seeded at 1×10⁵ cells/ml (0.5ml/well) in 8-Well Lab-Tek II Chamber Slides. After attachment overnight, PHA was administrated to cell culture at different doses: 0, 2.5, 5, 10, 20µg/ml in 0.5ml culture medium/well. Supernatants were collected after treatment for 3-5 days for examination of NO production [Ziche, M., L. Morbidelli, E. Masini, S. Amerini, H. J. Granger, C. A. Maggi, P. Geppetti, and F. Ledda. 1994. Nitric oxide mediates angiogenesis in vivo and endothelial cell growth and migration in vitro promoted by substance P. J Clin Invest. 94:2036-2044]. Absorbance was measured at 540 nm. Diluted Sodium nitrite (NaNO2, Sigma) solution was served as standard curve to calculate the amount of NO. The PHA-treated and untreated cells were used for iNOS immunostaining as described below.

For iNOS examination, immunostaining was performed as previously described with minor modifications [Zhao, Y., H. Wang, and T. Mazzone. 2006. Identification of stem cells from human umbilical cord blood with embryonic and hematopoietic characteristics. Exp Cell Res. 2006, 312: 2454-2464. DOI: 10.1016/j.yexcr.2006.04.008. April 26; (Epub ahead of print)]. In brief, PHA-treated and untreated cells were fixed with 4% paraformaldehyde for 20 min and then permeabilized with 0.5% Triton X-100 (Sigma) for 5-6 min at room temperature. After blocking endogenous perioxidase activity and non-specific binding, cells were incubated with rabbit anti-inducible nitric oxide synthase (iNOS) polyclonal antibody (Biomol International, Plymouth Meeting, PA). Then, cells were stained with ABC kit (Vector Laboratories, Burlingame, CA). Normal rabbit IgG (Santa Cruz) served as negative control. Cells were viewed and photographed using a Zeiss Axiocam Color Camera with Zeiss Axioskop Histology/Digital Fluorescence microscope.

### Regulation of CB-SC on CD4⁺, CD8⁺ T lymphocytes and CD4/CD8 ratio

To evaluate immune regulation of CB-SC on T cell subsets, CB-SC were initially cocultured with unsorted lymphocytes in the presence of different stimulators including IL-2 and PHA. Compared with lymphocytes alone, results showed that PHA stimulation could significantly increase the percentage of CD8⁺T cells and decrease CD4⁺T cell percentage by more than 2-fold respectively (*p*<0.05), with no changes on CD4⁻CD8⁻ T cells (Table 1); IL-2 stimulation could increase the percentage of CD4⁻CD8⁻ T cells, but failed to affect CD4/CD8 ratio. After coculture with CB-SC, the percentage of CD4⁻CD8⁻ T cells was significantly improved in both IL-2 and PHA stimulation (*p*< 0.05 and *P*<0.01, respectively); the percentage of CD8⁺ T-cell in PHA stimulation was reduced to control level by coculture with CB-SC, the decreasing of CD4⁺ T cell percentage was significantly reversed, and therefore CD4/CD8 ratio was significantly upregulated (*p*<0.05) (Table 1).

Above results suggest that CB-SC may display negative regulation on IL-2- or PHA-activated T cells. To date, increasing evidence demonstrate that CD69 molecule function as an important negative regulator on activated lymphocytes [Sancho D, Gomez M, Sanchez-Madrid F. CD69 is an immunoregulatory molecule induced following activation. Trends Immunol, 2005, 26:136-140]. To further evaluate effects of CB-SC on T cell subset at single cell level, we examined CD69 expression on the sorted CD4⁺ T cells and CD8⁺ T cells after coculture with CB-SC. Compared with lymphocyte control, CD69 expression was upregulated considerably in PHA-stimulated CD4⁺ and CD8⁺ T cells, only slightly upregulation in IL-2 stimulation (FIG.4 A and B). After coculture with CB-SC, however flow analysis demonstrated that CD69 expression was further significantly increased on both CD4⁺ T cells (FIG. 4A) and CD8⁺ T cells (Fig. 4B) in both IL-2- and PHA-activated lymphocytes (*p*<0.05). It indicates that CB-SC display immune regulation on both CD4⁺ and CD8⁺ T cell subsets.

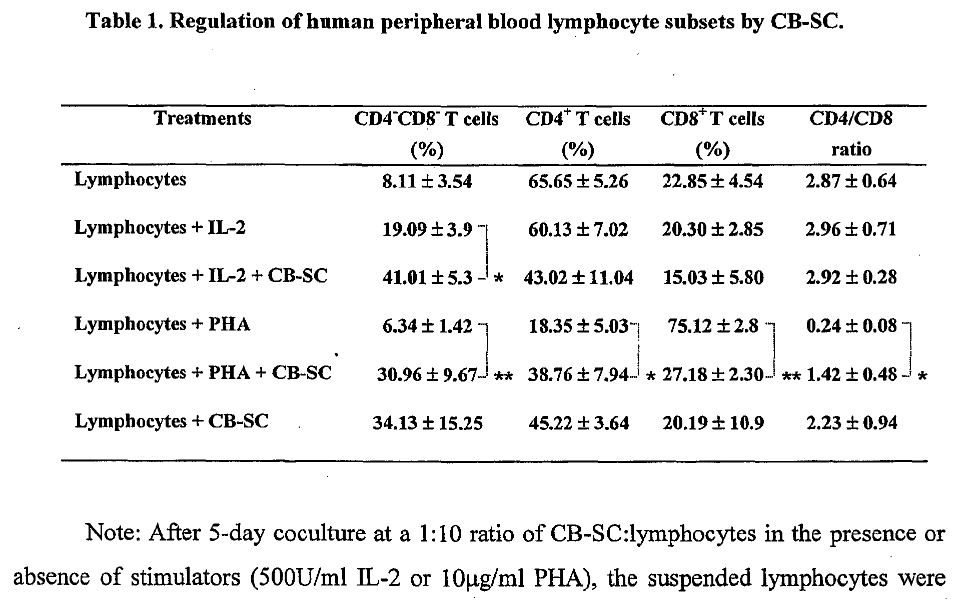

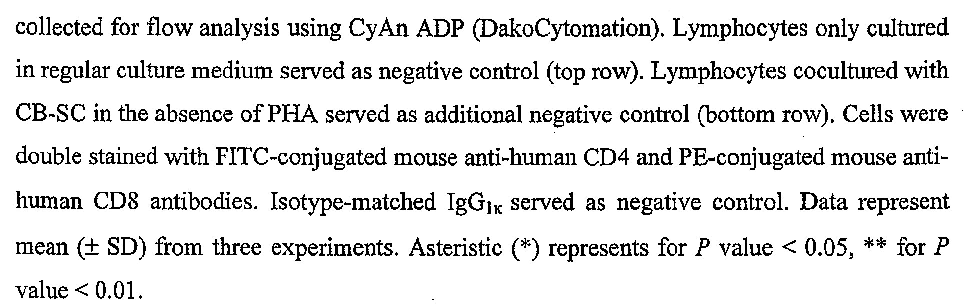

### Regulation of CB-SC on CD4⁺CD25⁺ regulatory T lymphocytes

Increasing evidence shows that regulatory T cells (Tregs) play a critical role in regulation of immune responses and homeostasis [Randolph, D.A. and C. G. Fathman. 2006. Cd4+Cd25+ regulatory T cells and their therapeutic potential. Annu Rev Med. 57: 381-402; Choileain, N.N. and H. P. Redmond. 2006. Regulatory T-cells and autoimmunity. J Surg Res. 130:124-135; Paust, S. and H. Cantor. 2005. Regulatory T cells and autoimmune disease. Immunol Rev. 204:195-207]. To evaluate effects of CB-SC on CD4⁺CD25⁺ Tregs, we performed cell sorting analysis using the sorted CD4⁺CD25⁺ Tregs from human peripheral blood. Results showed that IL-2 (500U/ml) could significantly stimulate the proliferation of CD4⁺CD25⁺ Tregs, but was inhibited after coculture with CB-SC. To evaluate action of cell-cell contacting, CB-SC were cocultured with IL-2-stimulated CD4⁺CD25⁺ Tregs presented in transwell system. Results showed that the proliferation of IL-2-stimulated CD4⁺CD25⁺ Tregs was reversed after CD4⁺CD25⁺ Tregs were separated from CB-SC (FIG.5). It suggests that cell-cell contacting plays a more important role than soluble factors on the inhibition of proliferation of IL-2-stimulated CD4⁺CD25⁺ Tregs.

### Regulation of CB-SC on interleukin-10 (IL-10) production

IL-10 plays a critical role in mediating immune regulation on Th1 and Th2 immune responses. Monocytes and B lymphocytes are the major source for human IL-10 [Moore, K.W., A. O'Garra, R. de Waal Malefyt, P. Vieira, and T. R. Mosmann. 1993. Interleukin-10. Annu Rev Immunol 11:165-190]. However, IL-10 is also produced other cell types including regulatory T cells [Hawrylowicz, C.M. and A. O'Garra. 2005. Potential role of interleukin-10-secreting regulatory T cells in allergy and asthma. Nat Rev Immunol. 5:271-83; Hawrylowicz, C.M. 2005. Regulatory T cells and IL-10 in allergic inflammation. J Exp Med. 202: 1459-1463; Battaglia, M., C. Gianfrani, S. Gregori, and M. G. Roncarolo. 2004. IL-10-producing T regulatory type 1 cells and oral tolerance. Ann N Y Acad Sci. 1029: 142-153], CD8⁺ T lymphocytes, mast cells, eosinophils, and keratinocytes [Moore, K.W., A. O'Garra, R. de Waal Malefyt, P. Vieira, and T. R. Mosmann. 1993. Interleukin-10. Annu Rev Immunol 11:165-190; Hawrylowicz, C.M. and A. O'Garra. 2005. Potential role of interleukin-10-secreting regulatory T cells in allergy and asthma. Nat Rev Immunol. 5:271-83; Hawrylowicz, C.M. 2005. Regulatory T cells and IL-10 in allergic inflammation. J Exp Med. 202: 1459-1463; Battaglia, M., C. Gianfrani, S. Gregori, and M. G. Roncarolo. 2004. IL-10-producing T regulatory type 1 cells and oral tolerance. Ann N Y Acad Sci. 1029: 142-153; Del Prete, G., M. De Carli, F. Almerigogna, M. G. Giudizi, R. Biagiotti, and S. Romagnani. 1993. Human IL-10 is produced by both type 1 helper (Th1) and type 2 helper (Th2) T cell clones and inhibits their antigen-specific proliferation and cytokine production. J Immunol 150: 353-360]. To evaluate IL10 production, we performed intracytoplasmic IL-10 analysis at single cell level using flow analysis. Results showed that 12% of untreated lymphocytes were positive for IL10; PHA stimulation could significantly enhance IL-10 production (FIG. 6). However, percentage of IL-10-positive cells in PHA-stimulated lymphocytes was decreased from 80% to 42% after coculture with CB-SC (FIG. 6).

### Inhibitory effects of CB-SC on PHA-stimulated lymphocyte proliferation and role of nitric oxide

Using phase-contrast microscopy, we observed that lymphocytes formed numbers of cell clumps of different size after stimulation with PHA in the absence of CB-SC (FIG. 7A, middle panel). However, the number of cell clumps was significantly reduced in the presence of CB-SC; most of lymphocytes were individually scattered in the culture medium and only a few cell clumps of very small size were observed (FIG. 7A, right panel). We further quantified cell number in different groups. Quantification of cell number showed a significant decrease in the PHA-stimulated lymphocyte proliferation by viable CB-SC (FIG. 7B, *p*<0.01). These results suggested that CB-SC could significantly inhibit the proliferation of PHA-stimulated allogeneic lymphocytes. To evaluate cell-to-cell contacting on this inhibition, we cocultured lymphocytes with 2.5% glutaraldehyde-fixed CB-SC. Results showed that lymphocyte proliferation was still significantly inhibited and but remained significantly higher in cocultured with the fixed CB-SC than that observed with viable CB-SC (FIG. 7B, *p*<0.02). These results suggest that both soluble factors and cell-to-cell contact participated in the inhibition of PHA-stimulated lymphocyte proliferation by CB-SC.

Next, we evaluated effects of CB-SC on physiological factor IL-2-stimulated lymphocyte proliferation. Using IL-2 (500U/ml) as potent stimulator can significantly stimulate lymphocyte proliferation. However, their proliferation were significantly inhibited after coculture with CB-SC at different ratios (FIG. 7C, *P<0.05*). It suggests that CB-SC could inhibit the proliferation of both PHA-and IL-2-stimulated lymphocytes.

To find which soluble factor participated in above process, we evaluated whether CB-SC expressed inducible nitric oxide synthase (iNOS) and produced NO in the presence of PHA. Immunostaining results initially demonstrated that CB-SC increased the expression of iNOS after treatment with PHA (FIG. 8A, right panel). PHA-untreated CB-SC showed background level of iNOS (FIG. 8A, left panel). Using Griess reaction [Y. Zhao, T. Mazzone, Human umbilical cord blood-derived f-macrophages retain pluripotentiality after thrombopoietin expansion, Exp Cell Res. 310 (2005) 311-318; Y. Hori, X. Gu, X. Xie, S. K. Kim, Differentiation of insulin-producing cells from human neural progenitor cells, PLoS Med. 2 (2005) 347-356], we examined NO production. Results showed that PHA-treated CB-SC produced NO in a dose-dependent manner (FIG. 8B). To confirm NO mediated the inhibitory effects, we administrated a powerful iNOS inhibitor L-NNA [Y. Zhao, T. Mazzone, Human umbilical cord blood-derived f-macrophages retain pluripotentiality after thrombopoietin expansion, Exp Cell Res. 310 (2005) 311-318 in the coculture of CB-SC with PHA-stimulated lymphocytes. The results demonstrated that cell number increased to a level about 4-fold higher in the presence of L-NNA than in the absence of L-NNA (FIG. 9, p<0.05). The data suggest that NO participated in inhibition of CB-SC on PHA-stimulated lymphocyte proliferation and could be partially blocked by administration of iNOS inhibitor (L-NNA).

### Example 8: CB-SC differentiation to endothelial-like cells

We used CB-SC cultured for 1-2 months for experiments examining cell differentiation. For differentiation to endothelial-like cells, CB-SC were treated with 50 ng/ml vascular endothelial growth factor (VEGF, R&D System, Minneapolis, MN) in RPMI 1640 medium supplemented with 7% fetal bovine serum and incubated at 37°C, 8% CO₂. After 10-14 days, VEGF-treated and -untreated CB-SC were examined for endothelial-associated markers.

Immunostaining results showed that ≈97% of VEGF-treated CB-SC expressed endothelial cell markers including Flt-1(VEGF receptor 1), Flk-1(VEGF receptor 2), von Willebrand Factor (vWF), and 76% of cells were positive for transmembrane glycoprotein CD146 (FIG. 10A, bottom of left panel). Untreated CB-SC did not express these antigens (FIG. 10A, top of left panel). Hematopoietic antigens including CD45 and CD117 were down regulated in VEGF-treated cells (FIG. 10A, right panel). In *vitro* functional analysis showed that both the VEGF-treated and untreated CB-SC possessed strong ability to incorporate acetylated low density lipoprotein (Ac-LDL) (FIG.10A). Additionally, the cellular morphology of VEGF-treated CB-SC changed to broad endothelial-like cells with spontaneous formation of chain-like structures (FIG. 10B). We further characterized these structures with specific endothelial cell marker CD146 and endocytosis of Ac-LDL and showed that they were double positive (Fig. 10C).

### Example 9: CB-SC differentiation to neuronall-like cells

Example 8 demonstrated that CB-SC produced endothelial-like cells, which arise from embryonic mesoderm [L. M. Hoffman, M.K. Carpenter, Characterization and culture of human embryonic stem cells, Nat Biotechnol. 23 (2005) 699-708; M. Baron, Induction of embryonic hematopoietic and endothelial stem/progenitor cells by hedgehog-mediated signals, Differentiation 68 (2001) 175-185]. We next evaluated the potential of CB-SC to differentiate to ectoderm-derived neuronal cells [L. M. Hoffman, M.K. Carpenter, Characterization and culture of human embryonic stem cells, Nat Biotechnol. 23 (2005) 699-708; L. Bally-Cuif, M. Hammerschmidt, Hammerschmidt. Induction and patterning of neuronal development, and its connection to cell cycle control, Curr Opin Neurobiol. 13 (2003) 16-25].

For differentiation to neuronal-like cells, CB-SC at 70% confluence were treated with 100ng/ml nerve growth factor (NGF, R&D System) in RPMI 1640 medium supplemented with 7% fetal bovine serum in 8-well Lab-Tek chamber slides (Nunc, Naperville, IL) and incubated at 37°C, 8% CO₂. After 10-14 days, NGF-treated and -untreated CB-SC were examined with neuronal markers.

Following the treatment of CB-SC with NGF, CB-SC displayed elongated and/or branched morphologies and formed neuronal-like net works through elongated cell processes (FIG. 11C). Immunostaining demonstrated they were positive for neuronal marker microtubule associated protein MAP-1B (FIG. 11C); untreated cells were negative or showed background staining (FIG. 11A). To further evaluate neuronal phenotypes, we examined NGF-treated cells for neuronal function-associated markers [C. Andressen, S. Arnhold, M. Puschmann, W. Bloch, J. Hescheler, R. Fassler, K. Addicks, Betal integrin deficiency impairs migration and differentiation of mouse embryonic stem cell derived neurons, Neurosci Lett. 251 (1998) 165-168]. As shown in FIG. 11C, 85% of cells were positive for synaptophysin; 78% of cells expressed neuronal transmitter γ-aminobutyric acid (GABA) along with its critical synthesizing enzyme glutamic acid decarboxylase (GAD). Untreated CB-SC cells were negative for these markers (FIG. 11A). Less than 5% of NGF-treated cells expressed dopaminergic neuron-associated tyrosine hydroxylase (TH) (data not shown). Hematopoietic antigens including CD9, CD45, and CD117 were down regulated following treatment with NGF (data not shown). Results suggest that NGF-treated CB-SC can give rise to GABAergic-like neurons.

### Example 10: In vivo differentiation of CB-SC to functional insulin-producing cells

The above examples demonstrated that CB-SC can differentiate to mesoderm-derived endothelial-like cells, along with ectoderm-derived neuronal-like cells in *vitro.* To provide additional evidence of CB-SC's differentiation potential, we investigated whether CB-SC can differentiate into endoderm-derived insulin-producing cells [M. A. Hussain, N. D. Theise, Stem-cell therapy for diabetes mellitus, Lancet 364 (2004) 203-205; L. M. Hoffman, M.K. Carpenter, Characterization and culture of human embryonic stem cells, Nat Biotechnol. 23 (2005) 699-708] *in vivo.*

Because we transplanted human stem cells, the immune-deficient mice must be used to avoid immune rejection. To date, there is not an ideal type 1 diabetic model (caused by autoimmune destruction) available for xenograft transplantation. We therefore performed in *vivo* transplantation of CB-SC into streptozotocin (STZ)-induced diabetic Balb/c nude mice and evaluated their capacity to correct hyperglycemia.

Diabetes in Balb/c nude male mice was induced with a single intraperitoneal injection of streptozotocin (STZ) (Sigma) 220 mg/kg of body weight, freshly dissolved in citrate buffer (pH = 4.5). Blood glucose levels were evaluated daily between 9 and 11 A.M. under nonfasting conditions. Diabetes was confirmed by the presence of weight loss, polyuria, and nonfasting blood glucose levels >350 mg/dl for 2 consecutive days. Diabetic mice were used for transplantation according to a protocol approved by the Animal Care Committee (ACC) of University of Illinois at Chicago. In brief, CB-SC at dosage of 5million cells/mouse in 0.5 ml physiological saline was injected into the peritoneal cavity by injection with 27-gauge needle, normally on day 3 following the injection of streptozotocin. The control mice were injected only with an equal volume of physisological saline. Blood glucose levels were monitored using an AccuChek glucose detector (Roche Diagnostics, Indianapolis, IN).

Seven days after transplantation, we performed intraperitoneal glucose tolerance testing (IPGTT). Mice (CB-SC-transplanted diabetic mice, untransplanted diabetic mice, and non-diabetic mice) were fasted overnight (12 h). Mice were weighed and injected intraperitoneally with a bolus of glucose (2 mg/g of body weight). Blood was then drawn from a tail vein at 0, 5, 10, 20, 30, 45, 60, 90, and 120 min after glucose administration. Glucose levels were measured from whole tail vein blood as described above. To measure human C-peptide, blood samples were collected from the tail vein during a 20 min time period before and following IPGTT. Blood human C-peptide level was detected by using an ultrasensitive human C-peptide enzyme-linked immunosorbent assay (ELISA) kit (Alpco Diagnostics, Windham, NH) following the manufacturer's protocols. This assay does not detect mouse C-peptide.

CB-SC-transplanted mice displayed significantly lower blood glucose levels (FIG. 12A). Further, intraperitoneal glucose tolerance testing (IPGTT) demonstrated physiological responses of transplanted CB-SC cells. Blood glucose of normal non-diabetic mice peaked between 5-10min and returned to normal level 30min following glucose challenge. Blood glucose of CB-SC-transplanted diabetic mice peaked at 20min, followed by a return to normal range after 60min (FIG. 12B). However, blood glucose of CB-SC-untransplanted diabetic mice remained very high (>500mg/dl) (FIG. 12B).

To substantiate that this reversal of hyperglycemia was associated with differentiation of CB-SC into insulin-producing cells, we performed the following experiments. Due to the cross reactivity of antibodies between human and mouse insulin, we utilized an assay that is specific for human C-peptide (a by-product of insulin production) to evaluate human insulin secretion [Y. Hori, X. Gu, X. Xie, S. K. Kim, Differentiation of insulin-producing cells from human neural progenitor cells, PLoS Med. 2 (2005) 347-356; A. Hayek, G. M. Beattie, Experimental transplantation of human fetal and adult pancreatic islets, J Clin Endocrinol Metab. 82 (1997) 2471-2475; M. Zalzman, S. Gupta, R. K. Giri, I. Berkovich, B. S. Sappal, O. Karnieli, M. A. Zern, N. Fleischer, S. Efrat, Reversal of hyperglycemia in mice by using human expandable insulin-producing cells differentiated from fetal liver progenitor cells, Proc Natl Acad Sci U S A. 100 (2003) 7253-8] from differentiated CB-SC cells. The results showed that human C-peptide was undetectable in mouse sera of CB-SC-untransplanted diabetic mice and normal non-diabetic mice, both prior to IPGTT and following IPGTT. In contrast, the human C-peptide level was significantly increased after IPGTT, to a level that was about 2 times higher than before IPGTT in the sera of the CB-SC-transplanted diabetic mice (p < 0.05) (FIG. 12C). These experiments demonstrated that CB-SC differentiated into functional insulin-producing cells in diabetic mice.

### Example 11: In vitro evidence for differentiation of CB-SC to insulin-producing cells

### Cord blood-derived stem cells (CB-SC) display nestin.

To date, nestin as an intermediate filament protein has been regarded as the marker of a neuroendocrine progenitor cells and nestin-positive cells can give rise to insulin-producing cells. Immunostaining showed that CB-SC strongly expressed nestin (FIG. 13).

### CB-SC display the potential to give rise to insulin-producing cells.

To evaluate potential of CB-SC differentiating to insulin-producing cells, we also examined pancreatic islet β-cell development-associated transcription factors, including PDX-1, NeuroD and NKX6.1. Western blot demonstrated that CB-SC strongly expressed NeuroD, PDX-1 (a well-known transcription factor essential for beta cell development), and NKX6.1 (that commits pancreatic progenitors to β cells) (FIG. 14). CB-SC also expressed prohormone convertase PC1 and PC2 (FIG. 14), which are usually presented in islet β cells and other cellular tissues associated with peptide synthesis.

### Expression of glucagon-like peptide 1 (GLP-1) receptor on CB-SC

Exendin-4, a long-acting agonist of GLP-1, can stimulate both beta cell differentiation and proliferation. Immunostainings have demonstrated around 76% of CB-SC expressed glucagon-like peptide 1 (GLP-1) receptor (FIG. 15). To optimize differentiation of CB-SC into insulin-producing cells and improve their therapeutic potential, therefore we have administrated exendin-4, in combination with high glucose and/or lipopolysaccharide (LPS).

### In vitro differentiation of CB-SC to insulin-producing cells.

In the presence of 10ng/ml exendin-4 + 50ng/ml lipopolyssacharide (LPS) + 25mM glucose, CB-SC could improve insulin production from 95.7 ± 29 to 288.5 ± 22 pg/mg cell protein, and C-peptide production from 4.3 ± 1.4 to 11.11 ± 2.7 fmol/mg cell protein.

### Example 12: Differentiation of CB-SC to oligodendrocytes

In the presence of nerve growth factor (NGF, 200ng/ml), we also found some of cells acquired oligodendrocytes-like morphology, with shorter branched cellular processes. Immunostaining results demonstrated that 25-29% of cells expressed specific oligodendrocyte markers myelin basic protein (MBP) (67-74), sulfatide 04, and galactocerebroside (Galc, a major glycolipid in mylin) (FIG. 16, right panel). However, no expression was observed in the NGF-untreated cells (FIG. 16, left panel).

### Example 13: Differentiation of CB-SC to megakaryocyte-like cells

We treated CB-SC with thrombopoietin (TPO, 10ng/ml). Cells were analyzed for megakaryocyte surface markers and their ploidy status. The results showed that around 70% of TPO-treated cells expressed megakaryocyte specific marker CD41b (FIG. 17). Dapi staining showed that these positive cells appeared with polyploidy nuclear. However, untreated cells failed to express these antigens with regular size of nuclear.

### Example 15: Expression of chemokine receptor CXCR4 on CB-SC.

Recently, increasing evidence demonstrate that the chemokine stromal cell-derived factor-1 (SDF-1) and its receptor, CXCR4, play an essential role in mediating hematopoietic stem cell homing. To evaluate the mechanism of CB-SC homing, we performed immunostaining analysis on the STZ-induced diabetic pancreatic islets. Results showed that diabetic pancreatic islets displayed SDF-1, but lack expression in pancreatic islets of normal NOD-scid mice (FIG. 18A). Notably, CB-SC expressed SDF-1 receptor CXCR4 (FIG. 18B). The data suggest that SDF-1/CXCR4 may contribute to guiding of CB-SC into pancreatic islets.

The practice of the present invention will employ and incorporate, unless otherwise indicated, conventional techniques of cell biology, cell culture, molecular biology, microbiology, genetic engineering, and immunology, which are within the skill of the art.

### REFERENCES

1. A. Vats, R. C. Bielby, N. S. Tolley, R. Nerem, J.M. Polak, Stem cells, Lancet 366 (2005) 592-602.
2. M. A. Hussain, N. D. Theise, Stem-cell therapy for diabetes mellitus, Lancet 364 (2004) 203-205.
3. C. M. Rice, N.J. Scolding, Adult stem cells--reprogramming neurological repair? Lancet 364 (2004) 193-199.
4. L. M. Hoffman, M.K. Carpenter, Characterization and culture of human embryonic stem cells, Nat Biotechnol. 23 (2005) 699-708.
5. K. H. Wilan, C.T. Scott, S. Herrera, Chasing a cellular fountain of youth, Nat Biotechnol. 23 (2005) 807-815.
6. C. Dennis, Check E, 'Ethical' routes to stem cells highlight political divide, Nature 437 (2005) 1076-1077.
7. M. Evans, Ethical sourcing of human embryonic stem cells--rational solutions? Nat Rev Mol Cell Biol. 6 (2005) 663-667.
8. D. A. Melton, G. Q. Daley, C. G. Jennings, Altered nuclear transfer in stem-cell research - a flawed proposal, N Engl J Med. 351 (2004) 2791-2792.
9. J. Bonde, D. A. Hess, J. A. Nolta, Recent advances in hematopoietic stem cell biology, Curr Opin Hematol. 11 (2004) 392-398.
10. K. K. Ballen, New trends in umbilical cord blood transplantation, Blood 105 (2005) 3786-3792.
11. P. R. Sanberg, A. E. Willing, S. Garbuzova-Davis, S. Saporta, G. Liu, C. D. Sanberg, P. C. Bickford, S. K. Klasko, N. El-Badri, Umbilical cord blood-derived stem cells and brain repair, Ann N Y Acad Sci. 1049 (2005) 67-83.
12. D. A. Peterson, Umbilical cord blood cells and brain stroke injury: bringing in fresh blood to address an old problem, J Clin Invest. 114 (2004) 312-314.
13. V. Silani, L. Cova, M. Corbo, A. Ciammola, E. Polli, Stem-cell therapy for amyotrophic lateral sclerosis, Lancet 364 (2004) 200-202.
14. K. Bieback, S. Kern, H. Kluter, H. Eichler, Critical parameters for the isolation of mesenchymal stem cells from umbilical cord blood, Stem Cells 22 (2004) 625-634.
15. E. J. Gang, S.H. Hong, J. A. Jeong, S. H. Hwang, S. W. Kim, I. H. Yang, C. Ahn, H. Han, H. Kim, In vitro mesengenic potential of human umbilical cord blood-derived mesenchymal stem cells, Biochem Biophys Res Commun. 321 (2004) 102-108.
16. G. Kogler, S. Sensken, J. A. Airey, T. Trapp, M. Muschen, N. Feldhahn, S. Liedtke, R. V. Sorg, J. Fischer, C. Rosenbaum, S. Greschat, A. Knipper, J. Bender, O. Degistirici, J. Gao, A. I. Caplan, E. J. Colletti, G. Almeida-Porada, H. W. Muller, E. Zanjani, P. Wernet, A new human somatic stem cell from placental cord blood with intrinsic pluripotent differentiation potential, J Exp Med. 200 (2004) 123-135.
17. Y. Zhao, T. Mazzone, Human umbilical cord blood-derived f-macrophages retain pluripotentiality after thrombopoietin expansion, Exp Cell Res. 310 (2005) 311-318.
18. C. P. McGuckin, N. Forraz, M. O. Baradez, S. Navran, J. Zhao, R. Urban, R. Tilton, L. Denner, Production of stem cells with embryonic characteristics from human umbilical cord blood, Cell Prolif. 38 (2005) 245-55.
19. H. J. Rippon, A. E. Bishop, Embryonic stem cells, Cell Prolif. 37 (2004) 23-34.
20. I. Klimanskaya, Y. Chung, L. Meisner, J. Johnson, M. D. West, R. Lanza, Human embryonic stem cells derived without feeder cells, Lancet 365 (2005) 1636-1641.
21. S. H. Orkin, Chipping away at the Embryonic Stem Cell Network, Cell 122 (2005) 828-830.
22. Y. Zhao, D. Glesne, E. Huberman, A human peripheral blood monocyte-derived subset acts as pluripotent stem cells, Proc Natl Acad Sci U S A 100 (2003) 2426-2431.
23. Drukker, G. Katz, A. Urbach, M. Schuldiner, G. Markel, J. Itskovitz-Eldor, B. Reubinoff, O. Mandelboim, N. Benvenisty, Characterization of the expression of MHC proteins in human embryonic stem cells, Proc Natl Acad Sci U S A. 99(2002) 9864-9869.
24. M. Baron, Induction of embryonic hematopoietic and endothelial stem/progenitor cells by hedgehog-mediated signals, Differentiation 68 (2001) 175-185.
25. L. Bally-Cuif, M. Hammerschmidt, Hammerschmidt. Induction and patterning of neuronal development, and its connection to cell cycle control, Curr Opin Neurobiol. 13 (2003) 16-25.
26. C. Andressen, S. Arnhold, M. Puschmann, W. Bloch, J. Hescheler, R. Fassler, K. Addicks, Betal integrin deficiency impairs migration and differentiation of mouse embryonic stem cell derived neurons, Neurosci Lett. 251 (1998) 165-168.
27. Y. Hori, X. Gu, X. Xie, S. K. Kim, Differentiation of insulin-producing cells from human neural progenitor cells, PLoS Med. 2 (2005) 347-356.
28. A. Hayek, G. M. Beattie, Experimental transplantation of human fetal and adult pancreatic islets, J Clin Endocrinol Metab. 82 (1997) 2471-2475.
29. M. Zalzman, S. Gupta, R. K. Giri, I. Berkovich, B. S. Sappal, O. Karnieli, M. A. Zern, N. Fleischer, S. Efrat, Reversal of hyperglycemia in mice by using human expandable insulin-producing cells differentiated from fetal liver progenitor cells, Proc Natl Acad Sci U S A. 100 (2003) 7253-8.
30. M. Richards, C. Y. Fong, W. K. Chan, P. C. Wong, A. Bongso, Human feeders support prolonged undifferentiated growth of human inner cell masses and embryonic stem cells, Nat Biotechnol. 20 (2002) 933-936.
31. M. Aoki, M. Yasutake, T. Murohara, Derivation of functional endothelial progenitor cells from human umbilical cord blood mononuclear cells isolated by a novel cell filtration device, Stem Cells 22 (2004) 994-1002.
32. D. A. Ingram, L.E. Mead, H. Tanaka, V. Meade, A. Fenoglio, K. Mortell, K. Pollok, M.J. Ferkowicz, D. Gilley, M.C. Yoder, Identification of a novel hierarchy of endothelial progenitor cells using human peripheral and umbilical cord blood, Blood 104 (2004) 2752-2760.
33. N. Baal, K. Reisinger, H. Jahr, R. M. Bohle, O. Liang, K. Munstedt, C. V. Rao, K. T. Preissner, M. T. Zygmunt, Expression of transcription factor Oct-4 and other embryonic genes in CD133 positive cells from human umbilical cord blood, Thromb Haemost. 92 (2004) 767-775.
34. M. Yu, Z. Xiao, L. Shen, L. Li, Mid-trimester fetal blood-derived adherent cells share characteristics similar to mesenchymal stem cells but full-term umbilical cord blood does not, Br J Haematol. 124 (2004) 666-675.
35. F. M. Cicuttini, K. Welch, A. W. Boyd, Characterization of CD34+HLA-DR-CD38+ and CD34+HLA-DR-CD38- progenitor cells from human umbilical cord blood, Growth Factors 10 (1994) 127-134.
36. G. K. Brolen, N. Heins, J. Edsbagge, H. Semb, Signals from the embryonic mouse pancreas induce differentiation of human embryonic stem cells into insulin-producing beta-cell-like cells, Diabetes 54 (2005) 2867-2874.
37. Y. Hori, I. C. Rulifson, B. C. Tsai, J. J. Heit, J. D. Cahoy, S. K. Kim, Growth inhibitors promote differentiation of insulin-producing tissue from embryonic stem cells, Proc Natl Acad Sci U S A. 99 (2002) 16105-16110.
38. H. Segev, B. Fishman, A. Ziskind, M. Shulman, J. Itskovitz-Eldor, Differentiation of human embryonic stem cells into insulin-producing clusters, Stem Cells 22 (2004) 265-274.
39. S. Miyazaki, E. Yamato, J. Miyazaki, Regulated expression of pdx-1 promotes in vitro differentiation of insulin-producing cells from embryonic stem cells, Diabetes 53(2004) 1030-1037.
40. M. Zalzman, L. Anker-Kitai, S. Efrat, Differentiation of human liver-derived, insulin-producing cells toward the beta-cell phenotype, Diabetes 54(2005) 2568-2575.
41. M. Zalzman, S. Gupta, R. K. Giri, I. Berkovich, B. S. Sappal, O. Karnieli, M. A. Zern, N. Fleischer, S. Efrat, Reversal of hyperglycemia in mice by using human expandable insulin-producing cells differentiated from fetal liver progenitor cells, Proc Natl Acad Sci U S A. 100(2003)7253-7258.
42. S. Yoshida, F. Ishikawa, N. Kawano, K. Shimoda, S. Nagafuchi, S. Shimoda, M. Yasukawa, T. Kanemaru, H. Ishibashi, L. D. Shultz, M. Harada, Human cord bloodderived cells generate insulin-producing cells in vivo, Stem Cells 23(2005) 1409-1416.
43. Zhao, Y., H. Wang, and T. Mazzone. 2006. Identification of stem cells from human umbilical cord blood with embryonic and hematopoietic characteristics. Exp Cell Res*.* DOI: 10.1016/j.yexcr.2006.04.008. April 26; (Epub ahead of print).
44. Ziche, M., L. Morbidelli, E. Masini, S. Amerini, H. J. Granger, C. A. Maggi, P. Geppetti, and F. Ledda. 1994. Nitric oxide mediates angiogenesis in vivo and endothelial cell growth and migration in vitro promoted by substance P. J Clin Invest. 94:2036-2044.
45. 18. Walter, U. and P. Santamaria. 2005. CD8+ T cells in autoimmunity. Curr Opin Immunol. 17:624-631.
46. Randolph, D.A. and C. G. Fathman. 2006. Cd4+Cd25+ regulatory T cells and their therapeutic potential. Annu Rev Med. 57: 381-402.
47. Choileain, N.N. and H. P. Redmond. 2006. Regulatory T-cells and autoimmunity. J Surg Res. 130:124-135.
48. Paust, S. and H. Cantor. 2005. Regulatory T cells and autoimmune disease. Immunol Rev. 204:195-207.
49. Moore, K.W., A. O'Garra, R. de Waal Malefyt, P. Vieira, and T. R. Mosmann. 1993. Interleukin-10. Annu Rev Immunol 11:165-190.
50. Hawrylowicz, C.M. and A. O'Garra. 2005. Potential role of interleukin-10-secreting regulatory T cells in allergy and asthma. Nat Rev Immunol. 5:271-83.
51. Hawrylowicz, C.M. 2005. Regulatory T cells and IL-10 in allergic inflammation. J Exp Med. 202: 1459-1463.
52. Del Prete, G., M. De Carli, F. Almerigogna, M. G. Giudizi, R. Biagiotti, and S. Romagnani. 1993. Human IL-10 is produced by both type 1 helper (Th1) and type 2 helper (Th2) T cell clones and inhibits their antigen-specific proliferation and cytokine production. J Immunol 150: 353- 360.
53. Sancho D, Gomez M, Sanchez-Madrid F. CD69 is an immunoregulatory molecule induced following activation. Trends Immunol. 2005, 26:136-140.

## Claims

1. Isolated embryonic-like stem cells from human umbilical-cord blood **characterized by**: (a) displaying embryonic stem cell characteristics comprising a capacity for proliferation and ability to differentiate to multiple types of cells; (b) displaying hematopoietic cell characteristics comprising CD45 expression; (c) phenotypically distinct from lymphocytes, macrophages and monocytes by being negative for the CD3, CD20, CD11b/Mac-1 and CD14 markers; (d) phenotypically distinct from hematopoietic stem cells by being negative for the CD34 marker; (e) displaying low immunogenicity by expressing major histocompatibility complex (MHC) molecules associated with low immunogenicity, and (f) displaying immune regulation by inhibition of mitogen-stimulated lymphocyte proliferation, for use in stem cell-based therapy.

2. The stem cells for use in stem cell-based therapy according to claim 1 wherein the stem cells are positive for Oct-4 and Nanog.

3. The stem cells for use in stem cell-based therapy according to any preceding claim wherein the stem cells are positive for CD9 .

4. The stem cells for use in stem cell-based therapy according to any preceding claim wherein the stem cells produce nitric oxide (NO).

5. The stem cells for use in stem cell-based therapy according to any preceding claim wherein the stem cells do not stimulate lymphocyte proliferation in an allogeneic mixed lymphocyte reaction.

6. The stem cells for use in stem cell-based therapy according to any preceding claim wherein the immune regulation is **characterized by** inhibitory effects of the stem cells on T lymphocyte proliferation and regulation on T cell subsets.

7. The stem cells for use in stem cell-based therapy according to any preceding claim capable of differentiating into at least one cell type selected from the group of endothelial-like cells, neuronal-like cells, insulin-producing cells, oligodendrocytes and megakaryocytes.

8. The stem cells for use in stem cell-based therapy according to any preceding claim for use in treating Parkinson's disease, diabetes, spinal cord damage, multiple sclerosis (MS) cardiovascular disease, stroke, birth defects or hyperglycemia.

9. A composition for use in stem cell-based therapy, comprising the isolated embryonic-like stem cells according to any one of claims 1-7.

10. A method for isolating the stem cells of any one of claims 1-8, comprising (a) providing a sample of human umbilical cord blood; (b) removing red cells from the sample to obtain mononuclear cells; (c) culturing the mononuclear cells in a culture medium in a non-tissue culture treated culture vessel; and (d) obtaining a cell population which is attached to the culture vessel.

11. A method of regulating lymphocyte subsets in a mixed T cell culture, the method comprising coculturing a diverse population of lymphocytes with the stem cells of any one of claims 1-7.

12. The method of claim 11 further comprising at least one of (a) increasing the CD4⁻CD8⁻ cell percentage, (b) increasing the CD8⁺ cell percentage, (c) decreasing the CD4⁺ cell percentage or (d) decreasing the CD4/CD8 ratio.

## Patentansprüche

1. Isolierte embryonal-ähnliche Stammzellen aus menschlichem Nabelschnurblut, **dadurch gekennzeichnet, dass** die Zellen:
(a) embryonale Stammzelleigenschaften umfassend die Fähigkeit zur Proliferation und die Fähigkeit zur Differenzierung in verschiedene Zelltypen von aufweisen,
(b) Eigenschaften von blutbildenden Zellen umfassend die Expression von CD45 aufweisen,
(c) phenotypisch unterschiedlich von Lymphozyten, Makrophagen und Monozyten durch negativ sein für CD3, CD20, CD11b/Mac-1 und CD14 Marker sind,
(d) phenotypisch unterschiedlich von blutbildenden Stammzellen durch negativ sein für den CD34 Marker sind,
(e) eine geringe Immunogenität durch Exprimieren von Haupthistokompatibititätskomplex- (MHC) Molekülen aufweisen, die mit einer geringen Immunogenität einhergehen, und
(f) eine Immunregulierung durch Inhibieren einer mitogenstimulierten Lymphozytenproliferation aufweisen,
zur Verwendung in einer auf Stammzellen basierenden Therapie.

2. Stammzellen zur Verwendung in einer auf Stammzellen basierenden Therapie nach Anspruch 1, worin die Stammzellen positiv für Oct-4 und Nanog sind.

3. Stammzellen zur Verwendung in einer auf Stammzellen basierenden Therapie nach einem der vorstehenden Ansprüche, worin die Stammzellen positiv für CD9 sind.

4. Stammzellen zur Verwendung in einer auf Stammzellen basierenden Therapie nach einem der vorstehenden Ansprüche, worin die Stammzellen Stickstoffmonoxid (NO) bilden.

5. Stammzellen zur Verwendung in einer auf Stammzellen basierenden Therapie nach einem der vorstehenden Ansprüche, worin die Stammzellen keine Lymphozytenprolifertaion in einer allogenen gemischten Lymphozytenreaktion stimulieren.

6. Stammzellen zur Verwendung in einer auf Stammzellen basierenden Therapie nach einem der vorstehenden Ansprüche, worin die Immunregulierung durch inhibitorische Wirkungen der Stammzellen auf eine T Lymphozytenproliferation und Regulierung von T Zellen Subtypen gekennzeichnet ist.

7. Stammzellen zur Verwendung in einer auf Stammzellen basierenden Therapie nach einem der vorstehenden Ansprüche, worin die Stammzellen fähig sind, in wenigstens einen Zelltyp ausgewählt aus der Gruppe aus endothelähnlichen Zellen, neuronenähnlichen Zellen, insulinproduzierenden Zellen, Oligodendrozyten und Megakaryozyten zu differenzieren.

8. Stammzellen zur Verwendung in einer auf Stammzellen basierenden Therapie nach einem der vorstehenden Ansprüche zur Verwendung in der Behandlung von Parkinson, Diabetes, Rückenmarkverletzungen, multipler Sklerose (MS), kardiovaskulären Erkrankungen, Schlaganfall, Geburtsschäden oder Hyperglykämie,

9. Zusammensetzung zur Verwendung in einer auf Stammzellen basierenden Therapie, umfassend die embryonal-ähnlichen Stammzellen nach einem der Ansprüche 1 bis 7.

10. Verfahren zum Isolieren der Stammzellen nach einem der Ansprüche 1 bis 8, umfassend
(a) Bereitstellen einer Probe von menschlichem Nabelschnurblut,
(b) Entfernen von roten Zellen aus der Probe um einkernige Zellen zu erhalten,
(c) Kultivieren der einkernigen Zellen in einem Kulturmedium in einem Kulturgefäß, das nicht mit einer Gewebekultur behandelt wurde, und
(d) Erhalten einer Zellpopulation, die an dem Kulturgefäß anhaftet.

11. Verfahren zum Regulieren von Lymphozyten Subtypen in einer gemischten T Zellkultur, wobei das Verfahren ein Nebenherkultivieren einer unterschiedlichen Population von Lymphozyten mit den Stammzellen nach einem der Ansprüche 1 bis 7 umfasst.

12. Verfahren nach Anspruch 11, ferner umfassend wenigstens einen der Schritte von
(a) Erhöhen des prozentualen CD4⁻CD8⁻ Zellanteils,
(b) Erhöhen des prozentualen CD8⁺ Zellanteils,
(c) Verringern des prozentualen CD4⁺ Zellanteils, oder
(d) Verringern des CD4/CD8 Verhältnisses.

## Revendications

1. Cellules souches de type embryonnaire isolées dérivées du sang de cordon ombilical humain, **caractérisées par** : (a) le fait de présenter des caractéristiques de cellules souches embryonnaires comprenant une capacité de prolifération et une capacité à se différencier en types de cellules multiples ; (b) le fait de présenter des caractéristiques de cellules hématopoïétiques comprenant l'expression de CD45 ; (c) le fait d'être distinctes d'un point de vue phénotypique des lymphocytes, des macrophages et des monocytes en étant négatives aux marqueurs CD3, CD20, CD11b/Mac-1 et CD14 ; (d) le fait d'être distinctes d'un point de vue phénotypique des cellules souches hématopoïétiques en étant négatives au marqueur CD34 ; (e) le fait de présenter une faible immunogénicité en exprimant des molécules du complexe d'histocompatibilité majeur (MHC) associées à une faible immunogénicité et (f) le fait de présenter une régulation immunitaire par l'inhibition de la prolifération lymphocytaire stimulée par des mitogènes, pour leur utilisation dans un traitement à base de cellules souches.

2. Cellules souches pour leur utilisation dans un traitement à base de cellules souches selon la revendication 1, dans lesquelles les cellules souches sont positives à Oct-4 et Nanog.

3. Cellules souches pour leur utilisation dans un traitement à base de cellules souches selon l'une quelconque des revendications précédentes, dans lesquelles les cellules souches sont positives à CD9.

4. Cellules souches pour leur utilisation dans un traitement à base de cellules souches selon l'une quelconque des revendications précédentes, dans lesquelles les cellules souches produisent de l'oxyde nitrique (ON).

5. Cellules souches pour leur utilisation dans un traitement à base de cellules souches selon l'une quelconque des revendications précédentes, dans lesquelles les cellules souches ne stimulent pas la prolifération lymphocytaire dans une réaction lymphocytaire mixte allogénique.

6. Cellules souches pour leur utilisation dans un traitement à base de cellules souches selon l'une quelconque des revendications précédentes, dans lesquelles la régulation immunitaire est **caractérisée par** des effets inhibiteurs des cellules souches sur la prolifération des lymphocytes T et la régulation de sous-groupes de lymphocytes T.

7. Cellules souches pour leur utilisation dans un traitement à base de cellules souches selon l'une quelconque des revendications précédentes, capables de se différencier en au moins un type de cellules choisies dans le groupe des cellules de type endothélial, des cellules de type neuronal, des cellules produisant de l'insuline, des oligodendrocytes et des mégacaryocytes.

8. Cellules souches pour leur utilisation dans un traitement à base de cellules souches selon l'une quelconque des revendications précédentes pour leur utilisation dans le traitement de la maladie de Parkinson, du diabète, des lésions de la moelle épinière, de la sclérose en plaques (SP), de maladies cardiovasculaires, de l'AVC, des anomalies congénitales ou de l'hyperglycémie.

9. Composition pour son utilisation dans un traitement à base de cellules souches, comprenant les cellules souches de type embryonnaire isolées selon l'une quelconque des revendications 1 à 7.

10. Procédé pour isoler les cellules souches selon l'une quelconque des revendications 1 à 8, comprenant (a) la fourniture d'un échantillon de sang de cordon ombilical humain ; (b) le retrait des érythrocytes de l'échantillon pour obtenir des cellules mononucléaires ; (c) la culture de cellules mononucléaires dans un milieu de culture dans un récipient de culture traité pour une culture non tissulaire ; et (d) l'obtention d'une population de cellules qui est fixée au récipient de culture.

11. Procédé de régulation de sous-groupes de lymphocytes dans une culture de lymphocytes T mixte, le procédé comprenant la co-culture d'une population diverse de lymphocytes avec les cellules souches selon l'une quelconque des revendications 1 à 7.

12. Procédé selon la revendication 11, comprenant en outre au moins un élément parmi (a) l'augmentation du pourcentage de cellules CD4⁻CD8⁻, (b) l'augmentation du pourcentage de cellules CD8⁺, (c) la diminution du pourcentage de cellules CD4⁺ ou (d) la diminution du rapport de CD4/CD8.
